# EUROPEAN PATENT SPECIFICATION

(11) **EP 4 031 117 B1**
(45) Date of publication and mention of the grant of the patent: **24.07.2024**
(21) Application number: 20775227.0
(22) Date of filing: 15.09.2020
(51) Int. Cl.: A61K 31/00, A61K 38/18, C07K 14/00, A61P 27/02

(54) **NGF MUTANT FOR USE IN TREATMENT OR PREVENTION OF OPHTHALMIC DISORDERS**
NGF-MUTANT ZUR VERWENDUNG IN DER BEHANDLUNG ODER PRÄVENTION VON AUGENERKRANKUNGEN
MUTANT DE NGF POUR UTILISATION DANS LE TRAITEMENT OU LA PRÉVENTION DE MALADIES OPHTALMIQUES

(30) Priority: 17.09.2019 EP 19197687
(43) Date of publication of application: 27.07.2022
(73) Proprietor: Chiesi Farmaceutici S.p.A., 43122 Parma (IT)
(72) Inventor: IMBIMBO, Bruno, Pietro, 43122 Parma (IT); VILLETTI, Gino, 43122 Parma (IT)
(74) Representative: Chiesi Farmaceutici S.p.A.
(86) International application number: PCT/EP2020/075697
(87) International publication number: WO 2021/052926

(56) References cited:
- WO-A1-2008/006893
- WO-A1-2013/092776
- FRANCESCA MALERBA ET AL: "Functional Characterization of Human ProNGF and NGF Mutants: Identification of NGF P61SR100E as a "Painless" Lead Investigational Candidate for Therapeutic Applications", PLOS ONE, vol. 10, no. 9, 15 September 2015 (2015-09-15), pages e0136425, XP055310595, DOI: 10.1371/journal.pone.0136425
- Y. BAI ET AL: "Chronic and Acute Models of Retinal Neurodegeneration TrkA Activity Are Neuroprotective whereas p75NTR Activity Is Neurotoxic through a Paracrine Mechanism", JOURNAL OF BIOLOGICAL CHEMISTRY, vol. 285, no. 50, 13 October 2010 (2010-10-13), US, pages 39392 - 39400, XP055340264, ISSN: 0021-9258, DOI: 10.1074/jbc.M110.147801
- SALLY L. ELSHAER ET AL: "Pronerve Growth Factor Induces Angiogenesis via Activation of TrkA: Possible Role in Proliferative Diabetic Retinopathy", JOURNAL OF DIABETES RESEARCH, vol. 2013, 1 January 2013 (2013-01-01), pages 1 - 10, XP055662833, ISSN: 2314-6745, DOI: 10.1155/2013/432659

## Description

### INTRODUCTION

### FIELD OF THE INVENTION

The present invention provides an agent suitable for the use in treatment and prevention of ophthalmic disorders, including but not limited to disorders involving a damage and/or disorder of the optic nerve and retina. The invention is defined by the appended claims.

### BACKGROUND OF THE INVENTION

The optic nerve is comprised of a bundle of more than 1 million nerve fibers that carry visual messages. The optic nerve connects retina to the brain. This connection comprises, at cellular level, retinal ganglion cells (RCGs). A retinal ganglion cell (RGC) is a type of neuron located near the inner surface (the ganglion cell layer) of the retina of the eye. RGCs vary in terms of their size, connections, and responses to visual stimulation, but they all share a long axon that extends into the brain. These axons form the optic nerve.

Damage to the optic nerve may cause vision loss. The type of vision loss and how severe it is, depends on where the damage occurs. It may affect one or both eyes.

There are many different types of optic nerve disorders including:
- Glaucoma is a group of diseases that are the leading causes of blindness inter alia in the United States. Glaucoma usually happens when the fluid pressure inside the eyes slowly rises and damages the optic nerve.
- Optic neuritis is an inflammation of the optic nerve. Causes include infections and immune-related illnesses such as multiple sclerosis. Sometimes the cause is unknown.
- Optic nerve atrophy is damage to the optic nerve. Causes include poor blood flow to the eye, disease, trauma or exposure to toxic substances or induced by drugs [ethambutol, isoniazid, digitalis, antibiotics (chloramphenicol, sulphonamides) and amiodarone].
- Optic nerve head drusen are pockets of protein and calcium salts that build up in the optic nerve over time.

All the above reported disorders may result in injury to the axon of the RGCs in the optic nerve that may ultimately lead to the death of these cells. For most of the optic nerve disorders, there is no treatment available, or treatment may only prevent further vision loss. Therefore, enhancing RGCs viability or function remains a major goal of basic and translational research.

So far, no treatment that is truly effective for counteracting the loss of RCGs in ophthalmic disorders has been brought to the market. Therefore, ophthalmic disorders, and particularly those that involve loss or damage of RCGs, have been so far difficult to treat. Therefore, there is still today a very high medical need.

Some effects of ocular administration in an experimental model of optic nerve injury have been described e.g. by Mesentier-Louro et al., 2019, Mol. Neurobiol., vol. 56, p. 1056-1069.

In rat models of optic nerve injury, massive RGCs degeneration generally occurs within 2 weeks following optic nerve crush as a consequence of reduced retro-transport of growth factors including nerve growth factor (NGF). According to the state of the art, intravitreal and eye drop administration of recombinant human NGF (rhNGF) might counteract optic nerve crush in adult rats only when administered immediately after optic nerve damage according to a preventive experimental paradigm (Mesentier-Louro et al., 2019, Mol. Neurobiol., vol. 56, p. 1056-1069). This does, however, not resemble the clinical situation of human ophthalmic disorders. For the intended therapy of human ophthalmic disorders, a therapeutic intervention can occur only after some lag time after inducing the first damage to the optic nerve, these experimental findings in the optic nerve crush model in the rat cannot be translated to clinical use in humans. In particular, as a result of the ONC further downstream processes could be triggered on the optic nerve, including potential further damaging of the RCGs. It remains unknown whether rhNGF or other molecules would be therapeutically effective when administered at a stage of post ONC damage of the RGC. It thus also remains elusive whether or not a therapy would be effective when an agent is administered in a situation including or resembling ophthalmic disorders in humans.

Thus, there is still a need for an effective treatment of eye conditions, particularly those affecting the optic nerve, which is not subject to adverse effects, such as intolerable or otherwise undesirable side effects, and for a therapeutic agent suitable for such purposes and available to practitioners at reliable and acceptable purity for administration to mammalian subjects, including humans.

WO 2008/006893 relates to muteins of hNGF, their therapeutic uses and pharmaceutical compositions. In particular, the document assesses the effects of NGF 61 Arg100Glu in a mouse model for Alzheimer's disease (Example 18) and measures the degree of hyperalgesia induced by NGF 61 Arg100Glu in CD1 mice (Example 19).

Malebra et Al. ("Functional Characterization of Human ProNGF and NGF Mutants: Identification of NGF P61SR100E as a "Painless" Lead Investigational Candidate for Therapeutic Applications", PLOS ONE, vol. 10, no. 9, 15 September 2015, page e0136425, DOI: 10.1371/journal.pone.0136425) characterize the mutant hNGF P61SR100E as a therapeutic candidate, through the assessment of *in vitro* stability by TF1 Proliferation assay; structural impact of the mutations by far-UV circular dichroism; responsiveness to the TrkA and p75 receptors in cellular models; algesic properties upon injection *in vivo* (page 27-28). The document discloses at p. 9, second paragraph, an NGF composition in which NGF P61SR100E is diluted in isotonic saline (NaCl 0.9 %).

Bai et Al. ("Chronic and Acute Models of Retinal Neurodegeneration TrkA Activity Are Neuroprotective whereas p75NTR Activity is Neurotoxic through a Paracrine Mechanism", JOURNAL OF BIOLOGICAL CHEMISTRY, vol. 285, no. 50, 13 October 2020, pages 39392-39400, DOI: 10.1074/jbc.M110.147801) studies the function of the TrkA and p76^{NTR} receptors and endogenous NGF and mutants NGF (NGF-KKE and NGF-Delta 9/13).

### PROBLEM TO BE SOLVED

It is a primary objective of the invention to provide a treatment or prevention for ophthalmic disorders, which is not associated with undesired or painful side effects, such as algesia. It is further desired to provide a therapeutic agent that can be readily used and administered by practitioners. It is also desired to provide a therapeutically active agent at sufficient yield and purity in order to enable such treatment. Thus, further objectives of the present invention include eliminating the disadvantages associated with the state of the art. These and other further objectives underlying the present invention will become apparent from the following detailed description, in light of the advantages achieved over the prior art. Particular objectives comprise the provision of a reliable method for treating s subject with an ophthalmic disorder without undesired side effects.

### SUMMARY OF THE INVENTION

The present invention provides a polypeptide for use in treatment and/or prevention of an ophthalmic disorder in a mammalian subject, wherein the ophthalmic disorder involves a damage and/or disorder of the optic nerve or a damage and/or disorder of retinal ganglion cells, and wherein the polypeptide is the polypeptide of SEQ ID NO: 4. Another polypeptide suitable for use in treatment and/or prevention of an ophthalmic disorder in a mammalian subject is the polypeptide of SEQ ID NO: 3, which is not part of the present invention. These polypeptides are characterized by a mutation of the amino acid sequence of human NGF (SEQ ID NO: 2), wherein said mutation is associated with reduced nociceptive activity. In particular, arginine at position 100 of hNGF is substituted by glutamic acid.

The polypeptide of SEQ ID NO: 4 is characterized by at least the absence of proline at position 61, more preferably by the substitution of proline at position 61 by another amino acid. In SEQ ID NO: 4, proline at position 61 of SEQ ID NO: 3 is substituted by serine.

Preferably, the mammalian subject is a human.

Preferably, the administration of the polypeptide of the invention is not causative for any undesired effects in the mammalian subject. It is specifically preferred that the treatment and/or prevention according to the present invention does not cause hyperalgesia in the mammalian subject.

Preferably, the polypeptide is administered following a damage of the optic nerve.

Preferably, the ophthalmic disorder comprises at least one selected from the group consisting of glaucoma, neurotrophic keratitis, optic neuritis, optic nerve atrophy, optic nerve head drusen and optic pathway glioma.

Preferably, the polypeptide is for administration to the eye. More preferably, administration is selected from the group consisting of topical administration to the eye and intravitreal administration, whereby topical administration to the eye is most preferred.

Preferably, the polypeptide is administered at least four days after induction of a damage of the optic nerve.

In one embodiment, the polypeptide is administered repeatedly. In a particularly preferred embodiment, the polypeptide is administered repeatedly at least three times per day.

In one embodiment, the polypeptide is administered repeatedly, e.g. until complete healing of the ophthalmic disorder, or at least until amelioration of the symptoms of the disorder is observed. Alternatively, the polypeptide is administered repeatedly for a period of three to 30 days, preferably seven to 14 days. Optionally, administration is discontinued after completion of said interval.

Preferably, the dose/each dose has an amount of 0.3 to 30 µg of the polypeptide per eye, more preferably 1 to 10 µg of the polypeptide per eye, and most preferably 5 µg of the polypeptide per eye. More preferably, these dosages are specifically for topical administration to the eye.

In one embodiment, the polypeptide is comprised in an aqueous medium, and the aqueous medium is administered to the mammalian subject. More preferably, the polypeptide is comprised in a composition comprising the following:
a) 0.2 to 20 mg/ml of said polypeptide,
b) 5 to 100 mM sodium acetate buffer,
c) 5 to 100 mM methionine,
d) pH 5.0 to 6.0.

Most preferably, the polypeptide is comprised in a composition comprising the following:
a) 2 mg/ml of said polypeptide,
b) 20 mM mM sodium acetate buffer,
c) 20 mM methionine,
d) pH 5.5.

In one embodiment, the polypeptide is obtainable from a biological source. This may comprise purification, i.e. separation from other molecules, including other proteins, such as host cell proteins. Optionally, the polypeptide of SEQ ID NO: 3 (which is not part of the present invention) or the polypeptide of SEQ ID NO: 4 is obtainable in a process which comprises (re-)folding and/or chromatographic purification and/or protease digestion, and optionally adjustment to final protein concentration and/ preparation of a desired formulation. In one embodiment, the polypeptide is obtainable by recombinant expression and purification, wherein the purification comprises purification on a mixed mode stationary phase. Preferably, the polypeptide for use according to the present invention is essentially free of degradants of the polypeptide, in particular essentially free of the des-nona variant of the polypeptide.

Thus, the present invention also provides the polypeptide from a recombinant source and purified as described herein for use in a method of treatment of the human or animal body by therapy, as described herein.

It is also disclosed a composition comprising a polypeptide selected among the polypeptide of SEQ ID NO: 3 and the polypeptide of SEQ ID NO: 4, the composition not being part of the present invention, wherein the composition is characterized by a pH of pH 5.0 to 6.0 (preferably pH 5.5), and comprises the following:
a) 0.2 to 20 mg/ml of said polypeptide (preferably 2 mg/ml),
b) 5 to 100 mM sodium acetate buffer (preferably 20 mM),
c) 5 to 100 mM methionine (preferably 20 mM).

### DETAILED DISCLOSURE OF THE INVENTION

This specification in its entirety, together with the claims and the figures, discloses specific and/or preferred embodiments and variants of the individual features of the invention. The present invention also contemplates as particularly preferred embodiments those embodiments, which are generated by combining two or more of the specific and/or preferred embodiments and variants described herein for the present invention. Thus, the present disclosure also includes all of the entities, compounds, features, steps, methods or compositions referred to or indicated in this specification, individually or collectively, and any and all combinations or any two or more of said entities, compounds, features, steps, methods or compositions. Thus, unless specifically stated otherwise herein or the context requires otherwise, reference to a single entity, compound, feature, step, method or composition shall be taken to encompass one and a plurality (i.e. more than one, such as two or more, three or more or all) of those entities, compounds, features, steps, methods or compositions. Unless specifically stated otherwise or the context requires otherwise, each embodiment, aspect and example disclosed herein shall be taken to be applicable to, and combinable with, any other embodiment, aspect or example disclosed herein.

Generally, unless specifically defined otherwise, all technical and scientific terms used herein have the same meaning as commonly understood by one of ordinary skill in the art (e.g., in medicine, ophthalmology, neurology, genetics, molecular biology, gene expression, cell biology, cell culture, immunology, neurobiology, chromatography, protein chemistry, and biochemistry). Textbooks and review articles published e.g. in English typically define the meaning as commonly understood by a person of ordinary skill in the art.

The expression "and/or", e.g., "X and/or Y" shall be understood to mean either "X and Y" or "X or Y" and shall be taken to provide explicit disclosure of "and", of "or" and of both meanings ("and" or "or").

As used herein, unless specified otherwise, the terms "about", "ca." and "substantially" all mean approximately or nearly, and in the context of a numerical value or range set forth herein preferably designates +/- 10 %, more preferably +/- 5 %, around the numerical value or range recited or claimed.

Unless expressly specified otherwise, the word "comprise", or variations such as "comprises" or "comprising" is used in the context of the present document to indicate that further members may optionally be present in addition to the members of the list introduced by "comprising". It is, however, contemplated as a specific embodiment of the present invention that the term "comprising" encompasses the possibility of no further members being present, i.e. for the purpose of this embodiment "comprising" is to be understood as having the meaning of "consisting of".

Unless expressly specified otherwise, all indications of relative amounts regarding the present invention are made on a weight/weight basis. Indications of relative amounts of a component characterized by a generic term are meant to refer to the total amount of all specific variants or members covered by said generic term. If a certain component defined by a generic term is specified to be present in a certain relative amount, and if this component is further characterized to be a specific variant or member covered by the generic term, it is meant that no other variants or members covered by the generic term are additionally present such that the total relative amount of components covered by the generic term exceeds the specified relative amount; more preferably no other variants or members covered by the generic term are present at all.

All methods and processes described herein can be performed in any suitable order unless otherwise indicated herein or unless the context clearly dictates otherwise.

The term "agent" as used herein, unless specified otherwise, generally refers to a compound or composition, preferably to a compound. An agent is capable of producing an effect on a living organism and/or on a cell from a living organism or derived from a living organism, e.g. by acting on a cell and/or on body tissue, or in an environment. The physical state of an agent is not particularly limited and, unless specified otherwise, may be in the air, water, and/or solid state. The type of agent is not particularly limited, unless specified otherwise, and thus, an agent may be a chemical and/or a biomolecule such as a protein or a nucleic acid. Specific agents defined herein are useful in the present invention.

An "adverse effect", as used herein, is an undesired harmful effect resulting from an administration of an agent (a drug) to a subject. Adverse effects include, without limitation, morbidity, mortality, hyperalgesic syndrome, pain, alteration in body weight, levels of enzymes, loss of function, or any pathological change detected at the microscopic, macroscopic or physiological level. Adverse effects may cause a reversible or irreversible change, including an increase or decrease in the susceptibility of the individual to other chemicals, foods, or procedures, such as drug interactions.

As used herein, the terms "chromatography", "chromatographic" and the like generally refer to a technique suitable for the separation of a mixture, wherein the mixture is added to a non-liquid material called the "stationary phase" with the purpose to separate, at least partially, one or more constituents of the mixture. For that purpose, the stationary phase may be exposed to a fluid and/or the mixture may be dissolved in a fluid; said fluid contacted with the stationary phase may also referred to as "mobile phase". In general, any step that is "carried out by chromatography", as described herein, may synonymously referred to as a "chromatographic step".

The term "mobile phase" as used herein, has the meaning typically used in the art and can refer to all fluids brought in contact with the stationary phase during chromatography, i.e. to wash fluids as well as to fluids (mixtures) comprising a protein of interest, such as one or more of the proteins described herein. In the present invention, the mixture subjected to chromatography, as specified herein, typically comprises one or more proteins, such as in particular the proteins described herein, such as the polypeptides of SEQ ID NO: 3 or 4, a precursor of any of these, a protease, and/or host cell proteins (HCP).

A "stationary phase" typically comprises a typically comprises a base matrix, which is a water-insoluble material, usually in particle from or gel form, such as a resin. In many cases, including embodiments described herein, a stationary phase comprises a base matrix and a moiety that can bind to at least one component comprised in the mixture that is to be subjected to chromatography. The base matrix is normally a water-insoluble material, usually in particle from or gel form. Non-limiting examples of base matrices are sepharose and agarose, for example highly rigid agarose.

A "chromatographic step" as used herein, refers to the action of adding to a chromatography material (preferably a stationary phase) a liquid comprising at least one compound to be analyzed and/or to be purified, which is preferably a protein (and in the context of the present invention said protein is most preferably the polypeptide of SEQ ID NO: 3 or SEQ ID NO: 4), optionally washing the chromatography material with one or more wash solutions, and eluting said at least one compound. In that context, a process characterized by two chromatographic steps, for illustration, is characterized in that a liquid comprising at least one such compound to be analyzed and/or to be purified is added to a first chromatographic material, as above described, and, after elution therefrom, the liquid comprising at least one such compound is added to a second chromatographic material, from which it is also eluted, as above described. It is the aim of any "chromatographic step" that at least one component comprised in the mixture applied to a stationary phase, preferably in chromatography, binds to the stationary phase. Such compound may be one or more proteins described herein. The compound may be recovered from the stationary phase, e.g. by exchange of mobile phase and/or by continued exposure to the mobile phase over time.

The term "binds", when used with reference to chromatography, such as to describe the binding capacity of a stationary phase, is not particularly limited, but typically refers to non-covalent binding. Thus typically at least one component comprised in a mixture, such as at least one protein, binds non-covalently to the stationary phase. A chromatographic step optionally but preferably comprises the washing of the stationary phase to which the at least one component is bound. The at least one component may be at least one protein, such as at least one protein described herein.

The term "heterologous" as used herein describes something consisting of multiple different elements.

The terms "disulfide" and "disulfide bond" are used, in the context of the present invention, within the meaning commonly used in the art. In general, a "disulfide" refers to a functional group with the structure R-S-S-R'. The linkage is also called an "SS-bond" and is usually derived by the coupling of two thiol groups. Disulfide bonds in proteins are formed between the thiol groups of the cysteine residues by the process of oxidative folding; such a specific disulfide bond between the thiol groups of two cysteine residues can also be referred to as "disulfide bridge". Without wishing to be bound to a particular theory, it is normally understood in the art that, in In eukaryotic cells, disulfide bridges are formed in the lumen of the endoplasmic reticulum (and the mitochondrial intermembrane space) but not generally in the cytosol, and, regarding prokaryotes, disulfide bridges are formed in the periplasm (of respective organisms, particularly Gram-negative bacteria); disulfide bridges can also be found in proteins of the extracellular environment of both eukaryotic and prokaryotic cells.

The terms "express", "expressed" and "expression", "gene expression" and the like, as used herein, relate to the use of information from a gene in the synthesis of a functional gene product. Gene expression comprises at least the transcription, and optionally comprises one of more additional features, optionally selected from the open list comprising translation and post-translational modification. In the context of recombinant expression of a protein in a host cell, the term normally implies that the protein is produced by the host cell (in any compartment of the cell and/or secreted and/or incorporated in inclusion bodies), unless the context dictates otherwise.

The terms "eye disorder" and "ophthalmic disorder" are used interchangeably herein and comprise all disorders affecting the eye. Without limitation, all disorders involving a damage and/or malfunction of the optic nerve and/or retina are comprised by the meaning of these terms.

The term "heterologous" as used herein describes something consisting of multiple different elements or origins. For example, in a non-human host cell which comprises a human gene (or gene encoding a non-natural polypeptide, such as the polypeptide of the invention) said gene is "heterologous" to the cell, and the cell may be capable of "heterologous" expression of the respective gene. Heterologous gene expression can also be referred to as "recombinant".

The term "inclusion body" has the meaning typically used in the art and is meant to refer to aggregates or particles found in the cytosol or in the periplasm of a host cell; inclusion bodies typically comprise protein, such as, in particular, protein expressed recombinantly in the host cell. Without wishing to be bound to any particular theory, it is understood that in the field of recombinant expression, inclusion bodies typically contain the recombinantly expressed protein but relatively little host cell protein (HCP), ribosomal components or DNA/RNA fragments. Without wishing to be bound to any particular theory, it is understood that inclusion bodies typically comprise, at least in part, protein that is not properly folded (misfolded protein), in particular misfolded recombinantly expressed protein. It is understood that inclusion bodies typically comprise protein in a non-properly folded form, i.e. in the context of the present invention they typically comprise the polypeptide according to the present invention and/or a precursor thereof, in a non-properly folded form. The term "misfolded" generally describes a biomolecule, such as a nucleic acid or polypeptide, which is not on the native conformation, i.e. in a non-properly folded form

By "isolated" is meant material that is substantially or essentially free from components that normally accompany it in its native state. For example, an "isolated peptide" or "isolated protein", as used herein, refers to a peptide or protein, respectively, which has been purified from the cellular and extracellular environment, such as tissue, which surround it in a naturally-occurring state, e.g., from the cell in which it has been expressed, such as a host cell. In an alternative description, an "isolated peptide" or "isolated protein" and the like, as used herein, refer to *in vitro* isolation and/or purification of a peptide or protein, respectively, from its natural cellular environment, and from association with other components of the environment in which the peptide or protein normally resides. In another example, an "isolated cell", as used herein, refers to a cell, which has been purified from the cellular and extracellular environment, such as tissue or cell colonies, which surround it in a naturally-occurring state, e.g., a host cell which has been removed from the environment that is normally adjacent to the cell. In accordance with the above definition of the word "isolated", "to isolate", as used herein, is the verb that describes the activity to obtain "isolated" material, such as e.g. an isolated cell or an isolated peptide or protein.

The terms "multi" and "multiple" as used herein mean a multitude, i.e. any number of two or more.

The term "mutation", as used herein, refers to the alteration of the nucleotide sequence of the genome of an organism, virus, or extrachromosomal DNA or other genetic elements. The term also extends to mutations of an amino acid sequence, particularly the amino acid sequence of a gene that carries at least one (non-silent) mutation. Unless specified otherwise, a mutation of the nucleotide sequence is a permanent alteration. Mutations present in the germ line are normally inheritable. In general, a mutation of the nucleotide sequence can result in many different types of change in sequences: mutations in genes can either have no effect, alter the product of a gene, or prevent the gene from functioning properly or completely. Mutations can also be present in non-genic regions. Unless specified otherwise, the wild type sequence is used as a reference sequence to describe a mutation. Thus, for example, when it is said that a given mutant is characterized by mutation of position 100 of a polypeptide sequence, this indicates that at position 100 the mutant does not have the same amino acid residue as the wild type polypeptide. Specific types of mutations of a nucleotide sequence and/or an amino acid sequence include alterations such as deletions, substitutions, additions, insertions and splice variants. A "deletion" with respect to a nucleotide sequence refers to the absence of one or more nucleotide(s) in the nucleotide sequence. A "deletion" with respect to an amino acid sequence refers to the absence of one or more amino acid residue(s) in the polypeptide. An "addition" with respect to a nucleotide sequence refers to the presence of one or more additional nucleotide(s) in nucleotide sequence. An "addition" with respect to an amino acid sequence refers to the presence of one or more additional amino acid residue(s) in the related polypeptide. A "substitution" with respect to a nucleotide sequence refers to the replacement of one or more nucleotide(s) by (an) other nucleotide(s) in the nucleotide sequence. A "substitution" with respect to an amino acid sequence refers to the replacement of one or more amino acid residue(s) by (an) other amino acid residue(s) in the polypeptide. Additions, deletions and substitutions to a nucleotide sequence, such as to an open reading frame, may be 5' terminus, the 3' terminus, and/or internal. Additions, deletions and substitutions to a polypeptide, may be at the amino terminus, the carboxy terminus, and/or internal. An "insertion" with respect to a nucleotide sequence and/or a polypeptide sequence is an addition of one or more nucleotides, or one or more amino acid residues, respectively, specifically at an internal position of the respective sequence. The term "splice variant" is used to describe that the RNA encoding a polypeptide sequence is spliced differently from the respective wild type RNA, typically as a result of a mutation at nucleic acid level, usually resulting in a polypeptide translation product which is different from the wild type polypeptide. The term "splice variant" can be used not only with respect to the respective RNA, but also with respect to the respective template DNA sequence (typically genomic DNA) and with respect to the sequence of the polypeptide encoded by such RNA.

The term "mutant" is generally intended to refer to a nucleic acid sequence or amino acid sequence which is different from the wild type sequence. A mutant nucleic acid sequence or amino acid sequence thus has at least one mutation with respect to the respective wild type sequence. In cases where polymorphisms at the nucleic acid sequence exist which are, however, not reflected at the level of the respective encoded polypeptide (silent mutations, degeneracy of the genetic code), the term "mutant", on nucleic acid level, specifically refers only to those nucleic acid variants which encode a mutant polypeptide. Mutants can contain different combinations of mutations, alone or in combination, including more than one mutation and different types of mutations.

The term "nerve growth factor", abbreviated "NGF" or "beta-NGF" stands for a neurotrophic factor and neuropeptide involved in the regulation of growth, maintenance, proliferation, and survival of certain neurons and other cells, in accordance with the common meaning in the art (see e.g. Levi-Montalcini, 2004, Progress in Brain Research, vol. 146, p. 525-527). Unless the context dictates otherwise, the term nerve growth factor stand for wild-type NGF only and does not include the polypeptides of SEQ ID NO: 3 or 4. Wild-type NGF is the 2.5S, 26-kDa beta subunit obtainable form a NGF precursor, which is biologically active: wild-type NGF binds with at least two classes of receptors: the tropomyosine receptor kinase A (TrkA) and low-affinity NGF receptor (LNGFR/p75NTR). The term "NGF", unless specified otherwise, refers to NGF of any species, preferably mammalian species; however, human NGF is always preferred. "hNGF", as used herein stands for human NGF. Unless the context dictates otherwise, the terms "NGF" and "hNGF" refer to wild-type NGF, i.e. hNGF stands for wild-type NGF. The amino acid sequence of wild-type human NGF corresponds to positions 121-239 of SEQ ID NO: 1 (grey in Fig. 24). The sequences of non-human NGF are available, e.g., in the scientific literature, through sequence searches, such as BLAST, using positions 121-239 of SEQ ID NO: 1 as bait, and in public protein databases such as Swissprot.

The terms "NGF mutein" and "mutein of NGF", or, with reference to NGF "mutein thereof, are used herein interchangeably to refer to a polypeptide which is characterized by at least one mutation, compared to wild-type NGF, as further described in detail herein. The polypeptides of SEQ ID NO: 3 and SEQ ID NO: 4 are muteins of NGF. Preferably a mutein of NGF has 80 to 99.5 % sequence identity with NGF, particularly human NGF, more preferably a mutein has 90 to 99% sequence identity with NGF, particularly human NGF.

The terms "mature part" "mature portion", with reference to NGF, are used interchangeably with the term "beta-NGF" and refer to a polypeptide of NGF which is characterized in that it does not comprise the pro-peptide (and hence, of course, not the pre-pro-peptide) of NGF. In analogy, the term "mature part" is also used to refer to the polypeptides of SEQ ID NO: 3 or 4, as these polypeptides likewise do not comprise a pro-peptide (and hence, of course, not a pre-pro-peptide). Preferably, the mature part does also not comprise a C-terminal cleavable peptide encoded by the wild-type NGF open reading frame; such C-terminal cleavable peptide, in the case of human NGF, consists of the two amino acid residues "RA" (240 and 241 in SEQ ID NO: 1). More particularly, the mature part is obtainable, without limitation, by cleavage of a pro-NGF with the protease Furin (and with other proteases capable of precisely cleaving directly N-terminal of the first amino acid residue of NGF, or of the polypeptide of SEQ ID NO: 3 or 4, respectively. For example, the Furin cleavage site of human NGF, and of many orthologs, is well known to consist of the sequence R¹S²K³R⁴ (one letter amino acid code, sequences numbered from N to C terminus; boxed in Fig. 25)). In mature NGF, normally neither the Furin cleavage site nor any amino acid N-terminally of the Furin cleavage site is present. For illustration, the mature part of human NGF consists of the polypeptide represented by amino acid positions 122-239 of SEQ ID NO: 1. The mature part of non-human NGF may be identified, e.g. by sequence search and/or sequence analysis, wherein said mature part of human NGF is used for sequence alignment.

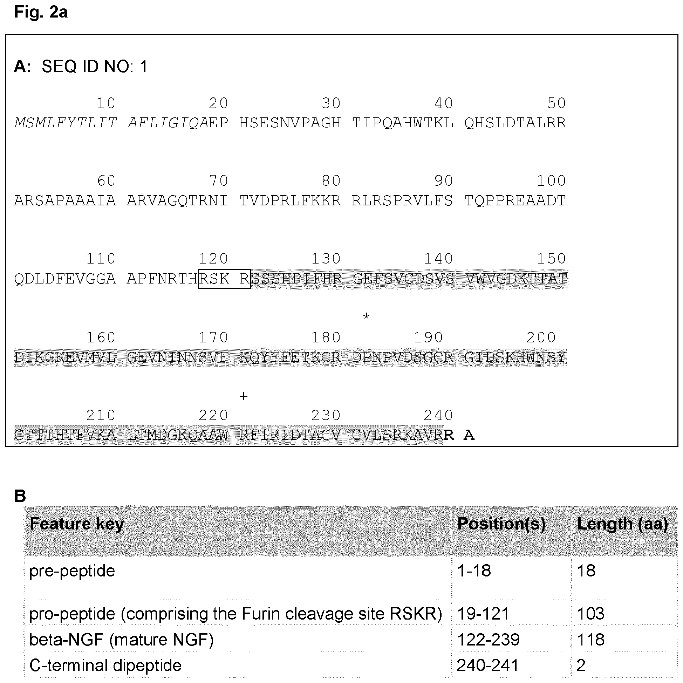

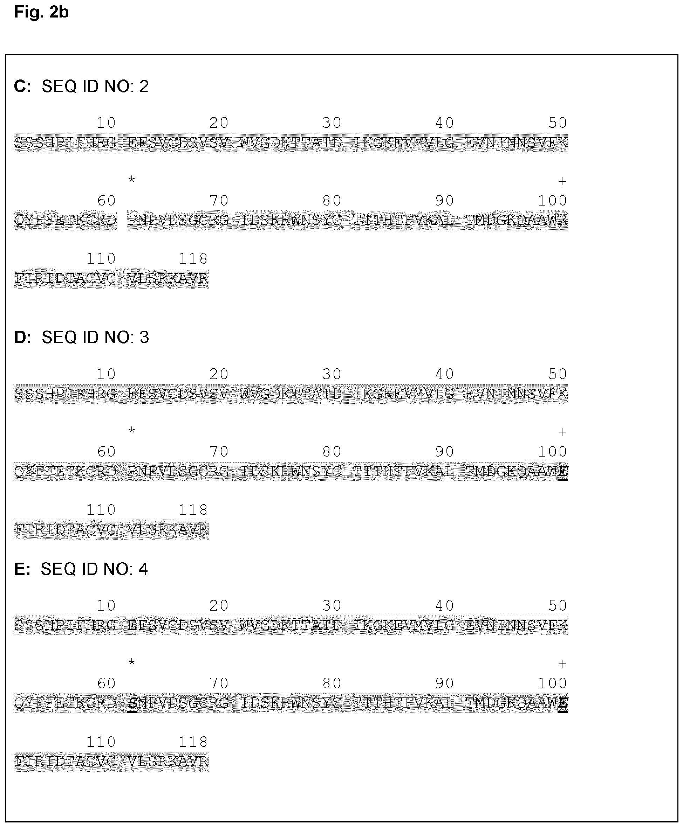

The terms "peptide" and "polypeptide" are used interchangeably herein and refer to a chain of amino acids linked by peptide (amide) bonds, and both terms include, without limitation, all the polypeptides as set forth in Fig. 2.

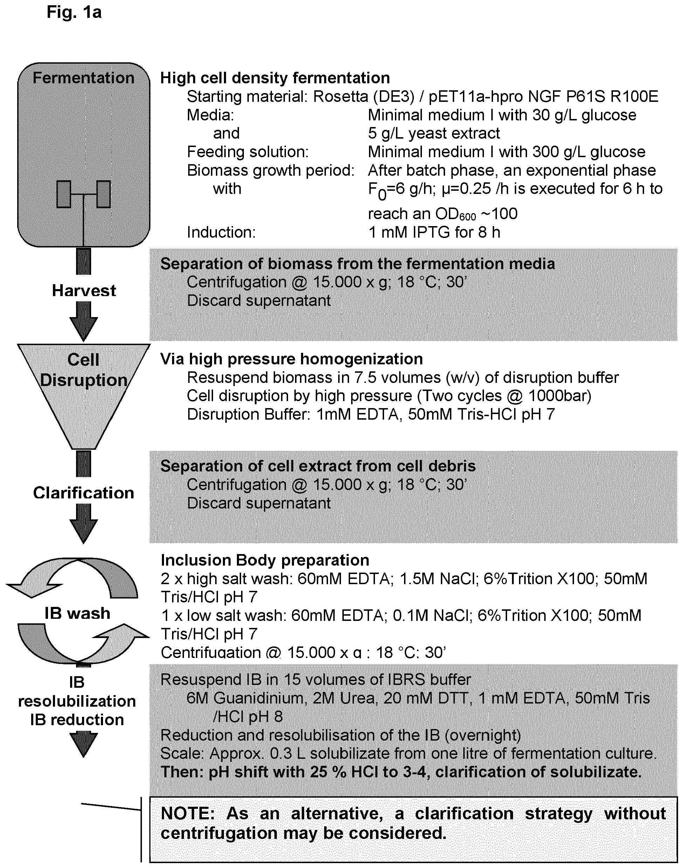

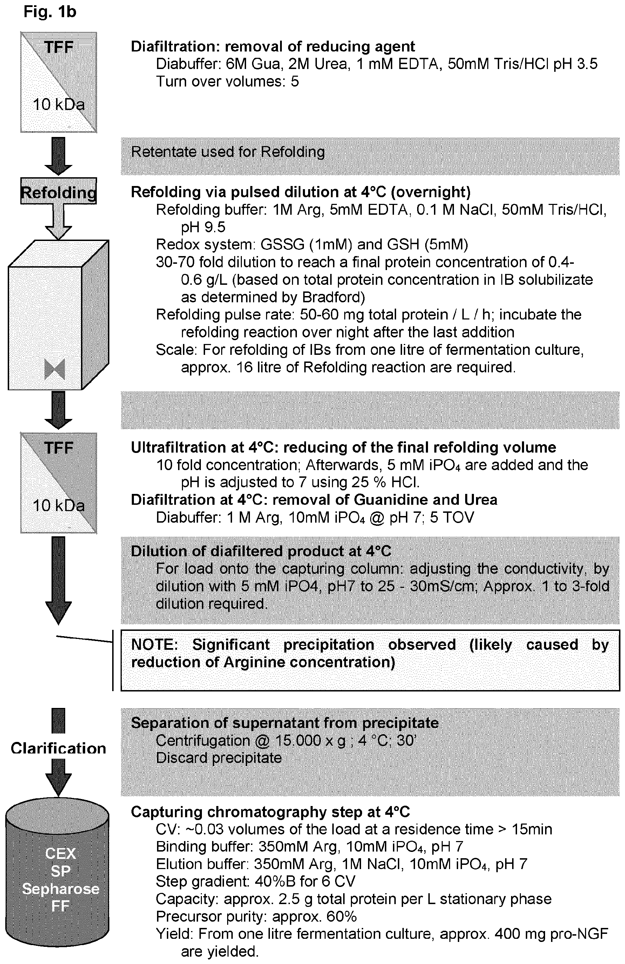

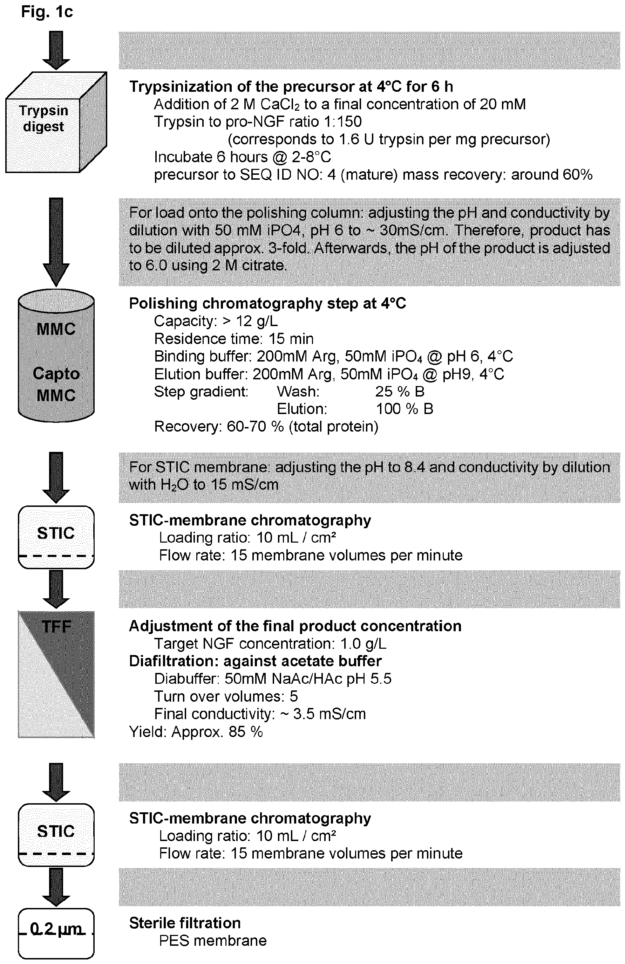

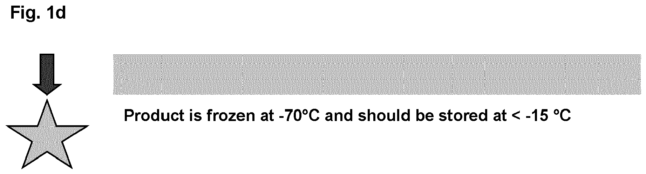

The term "precursor", as used herein with reference to NGF, refers to any peptide sequence from which NGF is obtainable through proteolytic cleavage. For illustration, both pro-NGF and pre-pro-NGF, as well as variants thereof, are typical examples of precursors of NGF. The term "precursor" as used herein, can refer to precursors the most C-terminal amino acid residue of which is the most C-terminal residue of NGF, and also to precursors which extend at the C-terminus beyond the most C-terminal residue of NGF, as long as NGF is obtainable therefrom by proteolytic cleavage: although the naturally occurring precursor of wild-type human pro-NGF (SEQID NO: 1) comprises a C-terminal dipeptide (amino acid residues 240 and 241 in SEQ ID NO: 1, bold in Fig. 1), it is preferable in the present invention that the precursor does not comprise a C-terminal cleavable peptide encoded by the wild-type NGF open reading frame; such C-terminal cleavable peptide, in the case of human NGF, consists of the two amino acid residues "RA" (240 and 241 in SEQ ID NO: 1.

The terms "pre-peptide" or "pre-sequence", as used herein, generally interchangeably refer to a polypeptide sequence encoded by part of the NGF open reading frame, N-terminally directly adjacent to the pro-peptide. For illustration: a pre-peptide is NGF consists of the sequence comprising the continuous sequence ranging from reside 1 of SEQ ID NO: 1 to residue 18 of SEQ ID NO: 1. The sequences of the respective pre-peptides of precursors of non-human NGF are available, e.g., in the scientific literature, through sequence searches, such as BLAST, using positions 1-18 of SEQ ID NO: 1 as bait, and in public protein databases such as Swissprot. A polypeptide or protein consisting of the pre-peptide and of pro-NGF, wherein the C-terminus of the pre-peptide is directly adjacent to the N-terminus of pro-NGF, can be referred to herein as "pre-pro-NGF" .

The terms "pro-peptide" or "pro-sequence", as used herein, generally interchangeably refer to a polypeptide sequence encoded in nature by part of the NGF open reading frame, N-terminally directly adjacent to mature NGF, but which polypeptide sequence does not include the pre-peptide. For illustration: a pro-peptide is comprised in the wild-type precursor of NGF. The pro-peptide of the precursor of NGF, consists of the sequence comprising the continuous sequence ranging from residue 19 of SEQ ID NO: 1 to residue 121 of SEQ ID NO: 1. The sequences of the respective pro-peptides of non-human pro-NGF are available, e.g., in the scientific literature, through sequence searches, such as BLAST, using positions 19-121 of SEQ ID NO: 1 as bait, and in public protein databases such as Swissprot.

"pro-NGF", as used herein, refers to a peptide sequence comprising both the mature part of NGF and the respective pro-peptide, but not the respective pre-peptide. Human pro-NGF consists of the sequence comprising the continuous sequence ranging from reside 19 of SEQ ID NO: 1 to at least residue 239 of SEQ ID NO: 1. Although wild-type human pro-NGF comprises a C-terminal dipeptide (amino acid residues 240 and 241 in SEQ ID NO: 1, bold in Fig. 25), it is preferable that the pro-NGF obtained and used in the present invention does not comprise a C-terminal cleavable peptide encoded by the wild-type NGF open reading frame; such C-terminal cleavable peptide, in the case of human NGF, consists of the two amino acid residues "RA" (240 and 241 in SEQ ID NO: 1). The sequences of non-human pro-NGF are available, e.g., in the scientific literature, through sequence searches, such as BLAST, using positions 19-239 of SEQ ID NO: 1 as bait, and in public protein databases such as Swissprot.

The terms "nucleic acid" and "polynucleotide" are used interchangeably herein, and refer to both RNA and DNA, including cDNA, genomic DNA, synthetic DNA, and DNA/RNA equivalents containing nucleotide analogs, phosphate analogs and/or sugar analogs. A nucleic acid can be double-stranded or single-stranded (i.e., a sense strand or an antisense strand). Non-limiting examples of polynucleotides include genes, open reading frames, gene fragments, exons, introns, messenger RNA (mRNA), transfer RNA, ribosomal RNA, siRNA, micro-RNA, ribozymes, cDNA, recombinant polynucleotides, branched polynucleotides, plasmids, vectors, isolated nucleic acids of any type and sequence nucleic acid probes, and primers, as well as nucleic acid analogs. Nucleic acids may have any type of three-dimensional structure.

The term "peptide" according to the invention comprises oligo- and polypeptides and refers to substances comprising two or more, preferably 3 or more, preferably 4 or more, preferably 6 or more, preferably 8 or more, preferably 10 or more, preferably 13 or more, preferably 16 more, preferably 21 or more and up to preferably 8, 10, 20, 30, 40 or 50, in particular 100 amino acids joined covalently to a chain by peptide bonds.

The term "protein" preferably refers to large peptides, preferably to peptides with more than 100 amino acid residues, but in general the terms "peptide", "polypeptide" and "protein" are synonyms and are used interchangeably herein, unless the context dictates otherwise. Thus, the terms "polypeptide of SEQ ID NO: 4 and "protein of SEQ ID NO: 4" have the identical meaning.

The term "pharmaceutically acceptable" generally describes that a certain substance can be administered to a subject, optionally and preferably in combination with an agent, without the agent causing intolerable adverse effects, at the dosage used.

The terms "pharmaceutically acceptable carrier" and "pharmaceutically acceptable excipient" are used to refer to any one or more of solvents, dispersion media, coatings, antibacterial and antifungal agents, isotonic and absorption delaying agents, and the like that are physiologically compatible and are suitable for administration to a subject as described herein, or do not otherwise interfere with such administration. Examples of such pharmaceutically acceptable carriers comprise without limitation one or more of water, saline, phosphate buffered saline, dextrose, glycerol, ethanol and the like, as well as combinations thereof. Particularly for the case of liquid pharmaceutical compositions, it may be preferable to include isotonic agents, for example, sugars, polyalcohols such as mannitol, sorbitol, or sodium chloride in the composition. Pharmaceutically acceptable carriers may further comprise auxiliary substances such as wetting or emulsifying agents, preservatives or buffers, which enhance the shelf life or effectiveness of the agent. A pharmaceutically acceptable carrier is typically comprised in a composition according to the present invention.

The term "pharmaceutically active agent" refers to an agent that can be used in the administration to a subject where the agent would be of benefit, e.g., in ameliorating the symptoms of a disease or disorder. In addition, a "pharmaceutically active agent" can have a positive or advantageous effect on the condition or disease state of a subject when administered to the subject in a therapeutically effective amount. Preferably, a pharmaceutically active agent has curative properties and may be administered to ameliorate, relieve, alleviate, reverse, delay onset of or lessen the severity of one or more symptoms of a disease or disorder. A pharmaceutically active agent may have prophylactic properties and may be used to delay the onset of a disease or to lessen the severity of such disease or pathological condition. For example, an agent of the invention is considered herein as a pharmaceutically active ingredient for the treatment of cystic fibrosis, as claimed. In another example, a pharmaceutically active protein can be used to treat a cell or an individual which does not normally express a protein, or not at the desired levels, or which mis-expresses a protein, e.g., a pharmaceutically active protein can compensate for a mutation, or for lack of sufficiently high expression, by supplying a desirable protein. The term "pharmaceutically active peptide or protein" includes entire proteins or polypeptides, and can also refer to pharmaceutically active fragments thereof. It can also include pharmaceutically active analogs of a peptide or protein.

An "open reading frame" or "ORF" is a continuous stretch of codons beginning with a start codon and ending with a stop codon.

The terms "subject" and "patient", as used herein, relate to a mammal. For example, mammals in the context of the present invention are humans, non-human primates, domesticated animals including but not limited to dogs, cats, sheep, cattle, goats, pigs, horses etc., laboratory animals including but not limited to mice, rats, rabbits, etc., as well as animals in captivity such as animals of zoos. The terms "subject" and "patient" as used herein particularly include humans. The subject (human or animal) has two sets of chromosomes; that is, the subject is diploid. The term "patient" refers to a subject which suffers from a condition, is at risk of suffering from a condition, has suffered from a condition, or is predicted to suffer from a condition, and which may be subjected to therapy, e.g. by administration of an agent. The patient's condition may be chronic and/or acute. Thus, a "patient" can also be described as a subject subjected to a therapy and/or or in need of a therapy.

The term "therapy" is to be understood broadly and refers to the treatment of a subject with the goal to prevent or treat a condition in the subject. In preferred embodiments, therapy specifically includes the administration of an agent to the subject.

The term "trypsin", as used herein, generally refers to a proteolytic enzyme classified as EC 3.4.21.4). Trypsin cleaves peptide chains mainly at the carboxyl side of the amino acids lysine or arginine, normally except when either is followed by proline. Without wishing to be bound by theory, it is understood that trypsin is a serine protease, and that trypsin is naturally found in the digestive system of many vertebrates, where it hydrolyzes proteins. Preferred in the present invention is trypsin from recombinant sources. Although, *in vivo,* trypsin is formed together with a pro-peptide (termed "trypsinogen"), the term "trypsin", as used herein preferably refers to mature trypsin devoid of any pro-peptide. The use of trypsin for proteolytic cleavage can also be referred to as "trypsin proteolysis" or "trypsinization", and proteins that result from cleavage with trypsin are said to have been "trypsinized".

A "variant" of a precursor of NGF or of the polypeptide of SEQ ID NO: 3 or 4, refers to a polypeptide or protein wherein the amino acid sequence that is not part of the mature NGF (beta-NGF) or not part of SEQ ID NO: 3 or 4, respectively, is characterized by at least one mutation in comparison with a wild-type precursor of NGF, such as with a wild-type pro-NGF or a wild-type pre-pro-NGF; said at least one mutation is preferably found N-terminal to the amino acid sequence of the mature NGF (beta-NGF). Thus, as used herein, a "variant" of a precursor of NGF or the like refers to a peptide or protein wherein the pre-peptide and/or the pro-peptide is characterized by at least one mutation, with respect to the amino acid sequence of the pre-peptide and/or the pro-peptide, for example but without limitation those variants described in WO 2013/092776 A1 and in by US 2018/0086805 A1. For illustration, WO 2013/092776 A1 describes "variants" of pro-NGF wherein the (wild-type) Furin cleavage site is absent due to one or more specific mutations.

The term "vector" or "cloning vector" generally refers to a nucleic acid that can be introduced into a host cell. Example vectors include, without limitation, plasmids, phages and all other types of nucleic acids that can be introduced into a host cell. The term "vector" is to be understood broadly and will comprise vectors which encode a peptide or protein for heterologous expression (such vectors may serve as templates, for the generation of transcripts), and those which do not. Vectors of the first type will contain an open reading frame encoding a protein or peptide, which may be expressed, when the vector is present in a host cell. Although the type of vector that the skilled person will choose will be dependent on the type of host cell that the skilled person will choose, in a particular case, cloning vectors for all common host cells, including *E. coli,* are commercially available, and the skilled person will thus choose a particular vector in full consideration of the host cell chosen.

The term "wild type" is used herein to refer to a gene or a protein typically found in nature, preferably in a healthy subject. A gene or a protein that is not "wild type" is referred to herein as "mutant" or "mutated", or the like. For illustration, SEQ ID NO: 1 shows the amino acid sequence of a precursor of wild-type human NGF; SEQ ID NO: 2 shows the amino acid sequence of wild-type human NGF.

The present invention is based on several findings, which are interrelated and thus together lead the inventors to arrive at the various aspects of the invention, which will all be described individually in the following.

### The agent according to the present invention

The present invention provides an agent for the treatment and/or prevention of ophthalmic disorder in a mammalian subject. The agent that can be used in the administration to a subject where the agent would be of benefit, e.g., in ameliorating the symptoms of a disease or disorder. In particular, the agent useful in the present invention is a polypeptide of SEQ ID NO: 4; another agent useful for the treatment and/or prevention of ophthalmic disorder in a mammalian subject is polypeptide of SEQ ID NO: 3, which is not part of the present invention. Thus, the present invention, in particular, provides a of SEQ ID NO: 4 for use in therapy. The therapy typically comprises administration of said polypeptide to a human or animal body, as described herein below.

The polypeptides of SEQ ID NO: 3 (which is not part of the present invention) and SEQ ID NO: 4 are provided as pharmaceutically active agents. The polypeptides of SEQ ID NO: 3 (which is not part of the present invention) or SEQ ID NO: 4 are provided for medical use, in particular for the treatment and/or prevention of ophthalmic disorder in a mammalian subject. Optionally, the polypeptides of SEQ ID NO: 3 or SEQ ID NO: 4 are from a recombinant source. Thus, the present disclosure provides also for the recombinant polypeptide of SEQ ID NO: 3 or SEQ ID NO: 4 for medical use, as described herein.

The agent according to the present invention, also termed herein "polypeptide of SEQ ID NO: 4" will now be described in more detail. The term "polypeptide of SEQ ID NO: 3" and similar terms denote herein a polypeptide comprising the amino acid sequence defined by SEQ ID NO: 3 and/or an agent with equivalent biological activity, which are not part of the present invention. The term "polypeptide of SEQ ID NO: 4" and similar terms denote herein a polypeptide comprising the amino acid sequence defined by SEQ ID NO: 4 and/or an agent with equivalent biological activity. Thus, within these terms are also included functionally equivalent parts or analogues of such polypeptides. One example of a biologically equivalent part of the polypeptide could be a domain or subsequence of the polypeptide of SEQ ID NO: 3 (not part of the present invention) or the polypeptide of SEQ ID NO: 4, which includes the binding site to enable the domain or subsequence to exert substantially the same biological activity as the full-length polypeptide of SEQ ID NO: 3 (not part of the present invention) or the full-length polypeptide of SEQ ID NO: 4 or alternatively a gene coding for such a polypeptide. The term "substantially the same biological activity" refers to an equivalent part or analogues polypeptide having at least 50%, preferably at least 60%, more preferably at least 70%, more preferably at least 75%, more preferably at least 80%, more preferably at least 85%, more preferably at least 90%, more preferably at least 95% and most preferably at least 97%, at least 98% or at least 99% of the activity of the polypeptide of SEQ ID NO: 3 (not part of the present invention) or the polypeptide of SEQ ID NO: 4 in the assays described in Example 4. An example of a biologically equivalent analogue of the polypeptide could be a fusion protein which includes at least a part of the amino acid sequence of the polypeptide of SEQ ID NO: 3 (not part of the present invention) or the polypeptide of SEQ ID NO: 4, but it can also be a homologous analogue of the polypeptide. Also, completely synthetic molecules that mimic the specific biological activity of the polypeptide of SEQ ID NO: 3 (not part of the present invention) or the polypeptide of SEQ ID NO: 4 would constitute "biologically equivalent analogues".

More preferably, the term "polypeptide of SEQ ID NO: 3" and similar terms denote herein a polypeptide comprising the amino acid sequence defined by SEQ ID NO: 3, which is not part of the present invention; such agents are optionally fusion proteins which comprise inter alia the amino acid sequence defined by SEQ ID NO: 3. Most preferably, the term "polypeptide of SEQ ID NO: 3" and similar terms denote herein a polypeptide consisting of the amino acid sequence defined by SEQ ID NO: 3; in this embodiment, the agent consists of a polypeptide consisting of the 118 amino acid residues in sequential order as defined by SEQ ID NO: 3. In this and other embodiments, this polypeptide optionally carries one or two or three internal cysteine bonds, so that cysteine (Cys, C) residues are covalently linked to each other to form intramolecular disulfide bridges. The cysteine bonds are preferably equivalent to those in wild-type human NGF.

Equally more preferably, the term "polypeptide of SEQ ID NO: 4" and similar terms denote herein a polypeptide comprising the amino acid sequence defined by SEQ ID NO: 4; such agents are optionally fusion proteins which comprise inter alia the amino acid sequence defined by SEQ ID NO: 4. Most preferably, the term "polypeptide of SEQ ID NO: 4" and similar terms denote herein a polypeptide consisting of the amino acid sequence defined by SEQ ID NO: 4; in this embodiment, the agent consists of a polypeptide consisting of the 118 amino acid residues in sequential order as defined by SEQ ID NO: 4. In this and other embodiments, the polypeptide optionally carries one or two or three internal cysteine bonds, so that cysteine (Cys, C) residues are covalently linked to each other to form intramolecular disulfide bridges. The cysteine bonds are preferably equivalent to those in wild-type human NGF.

The polypeptide of the present invention may optionally be characterized by further posttranslational modifications. Such posttranslational modifications optionally include glycosylation and/or phosphorylation. Preferably, however, the polypeptide according to the present invention is free of glycosylation and/or phosphorylation. Indeed, considering that the experimental examples herein demonstrate a beneficial effect on the healing of eye disorders and a beneficial benefit-to-adverse effect ratio, whereby the polypeptide used was obtained by cytosolic recombinant expression in bacteria, which typically does not result in glycosylation and/or phosphorylation, it is plausible that the beneficial effect of the present invention is not dependent on such type of posttranslational modification. Therefore, in preferred embodiments, the polypeptide according to the present invention is not characterized by glycosylation and/or phosphorylation.

Typically, the polypeptide according to the present invention is a non-natural polypeptide which is not naturally produced by the subject to which the polypeptide is administered. This is associated not only with the advantage of detectability in the subject post administration, but also evidences that administration (from an external source, such as e.g. the compositions prepared according to the present disclosure) needs to be administered to the subject in order to achieve success in treatment or prevention of the disorder.

Preferably the polypeptide according to the present invention is an isolated polypeptide. More preferably, the polypeptide according to the present invention is essentially free of host cell proteins, degradation products (such as des-nona variant, for example), and protease (such as trypsin, for example). When the polypeptide according to the present invention is essentially free of host cell proteins, degradation products (such as des-nona variant, for example), and protease (such as trypsin, for example) is may also be referred to as "pure polypeptide". Preferably, the polypeptide according to the present invention is administered as pure polypeptide. More preferably, the pure polypeptide consisting of SEQ ID NO: 3 (not part of the present invention) or the pure polypeptide consisting of SEQ ID NO: 4 has a weight percentage of 90 % or more, preferably 92 % or more, more preferably 93 % or more, more preferably 94 % or more, more preferably 96 % or more, more preferably 97 % or more, more preferably 98 % or more, more preferably 99 % or more, more preferably 99.2 % or more, more preferably 99.4 % or more, more preferably 99.6 % or more, more preferably 99.8 % or more, more preferably 99.9 % or more, with respect to the total protein in the composition. Such pure polypeptide is available based on the disclosure herein, including Examples 1 and 2. Most preferably, the pure polypeptide according to the present invention has a purity grade compatible with Good manufacturing practices (GMP).

As demonstrated in the experiments herein, particularly Example 4, the administered doses of the agent according to the present invention did not induce any hyperalgesic syndrome (pain) in separate experiments. The absence of pain is particularly remarkable because the agent was administered by topically, and repeatedly, to the damaged eye, also in a chronic setting (for details see Examples).

Optionally, the polypeptide of SEQ ID NO: 3 (not part of the invention) or of SEQ ID NO: 4 is administered in an effective amount to a subject in need thereof. Details of the administration, the effective amount and of the subject in need thereof are described herein below.

The polypeptides consisting of SEQ ID NO: 3 (not part of the invention) and of SEQ ID NO: 4, respectively, differ in one or two positions from the amino acid sequence of human nerve growth factor (NGF, also referred to as wild-type human NGF or wild-type NGF, see SEQ ID NO: 2). The difference of the polypeptide according to the present invention with respect to the polypeptide of SEQ ID NO: 2 has a remarkable effect on the treatment or prevention of eye disorders and the absence of side effects, as disclosed in detail herein and supported by the experimental examples herein.

Nerve growth factor (NGF) is a neurotrophin required for the development and survival of specific neuronal populations. NGF is a homodimeric peptide that naturally triggers proliferation and homeostasis of neurons. In the body, NGF binds with at least two types of receptors: the tropomyosine receptor kinase A (TrkA) and low-affinity NGF neurotrophin receptor p75 (LNGFR/p75^{NTR}/p75). Both are associated with certain disorders in humans and animals, although the respective mechanisms of action are likely different. Several therapeutic applications for NGF have been proposed but few have matured to the market.

However, many therapeutic uses of NGF which have been envisaged in the past have not matured to marketed therapeutic NGF products, and one reason can be seen in that NGF, besides the desired effect on proliferation and homeostasis of neurons, is associated with pain: it can, when administered topically or systemically, cause hyperalgesia (Lewin et al., 1994, Eur. J. Neurosci., vol. 6, p. 1903-1912; Della Seta et al., 1994, Pharmacol. Biochem. Behav., vol. 49, p. 701; Dyck et al, 1997, Neurology, vol. 48, 501-505; McArthur, et al., 2000, Neurology, vol. 54, p. 1080-1088 ; Svensson et al., 2003, Pain, vol. 104, p. 241-247 ; Ruiz et al., 2004, Brain Res., vol. 1011, p. 1-6). As a solution, mutant versions of NGF ("muteins") were developed, which are associated with reduced nociceptive activity ("painless NGF"), and which are characterized by at least one mutation in the domain of NGF which interacts with the TrkA receptor (WO 2008/006893 A1, Malerba et al. PLOS One, 2015, vol. 10, e0136425). However, such polypeptides are so far not available to the public in pharmaceutically acceptable purity, and have not been proposed or developed for the treatment or prevention of ophthalmic disorders of the eye, possibly also in view of the prejudice and general negative experience with research on growth factors in this therapeutic field in general.

It is known that (wild-type) NGF affects target cells through binding to two separate receptors, (a) receptor tyrosine kinase A (TrkA), which leads to neuronal survival, and (b) p75 neurotrophin receptor, which is involved in the regulation of cell death (Mesentier-Louro et al., 2017, Int. J. Mol. Sci., vol. 18(98), so that polypeptide sequence elements with wild-type NGF, following optic nerve crush, may also have the effect of exacerbation of retinal degeneration via stimulation of apoptosis, see Mesentier-Louro et al., 2018, Mol. Neurobiol., vol. 56, p. 1056-1069. It is also known that the residue R100 of wild-type human NGF is involved in binding of NGF to p75, and that mutation of that residue affects p75 binding, see e.g. WO 2008/006893 A1. In contrast to wild-type NGF, hNGF, the polypeptides of the present invention have a lower binding affinity for p75 (see e.g. Malerba et al., 2015, PlosOne, 10(9): e0136425). human NGF P61SR100E corresponds to the polypeptide of SEQ ID NO: 4. Among several mutants of human NGF (hNGF mutants), the mutant human NGF P61SR100E is considered as most promising and the mutants are mentioned to be suitable inter alia for ophthalmic diseases. However, the specific uses of treatment and/or prevention according to the present invention are not taught by those references, and those references also fail to make the respective polypeptides available at a high enough purity to enable it medical use in humans. Thus, the polypeptides for use according to the present invention, as described and provided herein, provide several unexpected advantages over wild-type human NGF. In particular, the experimental findings underlying the present invention (see Examples) cannot be explained solely by the fact that the agent according to the present is "painless", since its ability to induce pain has never been experimentally investigated in an innervated area of the eye, i.e. an area characterized by exposed nociceptors. Furthermore, even though administration of the agent according to the present invention is causative for nerve strengthening (see e.g. Example 3), the administration is not associated with pain.
the stability and thus the long-term purity of the polypeptide of SEQ ID NO: 3 (not part of the present invention) or SEQ ID NO: 4 thereof can be obtained and/or improved by the aspects and embodiments described herein. Thus, the present disclosure not only makes a new treatment or prevention for an ophthalmic disorder available, but also provides the agent suitable for such treatment or prevention, at a purity grade suitable for therapeutic applications, including administration to a mammal. The agent of the present invention was not previously available to the public at such advantageous purity grade.

The polypeptide of SEQ ID NO: 3 (not part of the invention) or SEQ ID NO: 4 is not found in nature and can also be referred to as a non-natural polypeptide. Thus, the agent according to the present invention is not wild-type NGF, and in particular not wild-type human NGF.

Preferably, the non-natural polypeptide according to the present invention is provided at high purity. Optionally, the polypeptide comprises internal disulfide bridges. Optionally, the polypeptide is properly folded. Optionally, the polypeptide is soluble in an aqueous medium.

The present invention is, in part, based on experiments with animal models of optic nerve damage or disorder (see Example 4). The polypeptide has induced a significant and dose-dependent improvement in the healing time of the disorders involving a damage and/or disorder of the optic nerve in comparison with placebo treated animals. This improvement is evident at doses devoid of pain-related side-effects thus demonstrating a potential benefit over the state of the art.

In particular, data generated in *in vivo* models of disorders involving a damage and/or disorder of the optic nerve have demonstrated that the polypeptide of the present invention is painless, yet retains the activity of targeting the NGF receptor system, and thereby provides as a therapeutic means for the treatment or prevention of ophthalmic disorders. Indeed, the polypeptide of the invention retains the trophic properties of wild-type NGF on angiogenesis and re-innervation that favors healing of the optic nerve without exerting the pro-nociceptive effects of wild-type NGF at the site of application and at the systemic level.

It is provided a polypeptide for use in treatment and/or prevention of an ophthalmic disorder in a mammalian subject, wherein the polypeptide is selected among the polypeptide of SEQ ID NO: 3 (not part of the invention) and the polypeptide of SEQ ID NO: 4. It is also provided the polypeptide of SEQ ID NO: 3 (not part of the invention) or SEQ ID NO: 4 for use in a method of treatment of the human or animal body by therapy, as described herein.

More particularly, the present invention relates to a specific therapeutic use of the polypeptide of SEQ ID NO: 4, wherein the specific therapeutic use is the treatment and/or prevention of an ophthalmic disorder in a mammalian subject. Thus, the present invention also provides the polypeptide of SEQ ID NO: 4 for use in a method of treatment of the human or animal body by therapy, wherein the therapy comprises the treatment and/or prevention of an ophthalmic disorder in a mammalian subject. The mammalian subject is typically a subject characterized by the need of such treatment.

The polypeptide of SEQ ID NO: 3 as well as the polypeptide of SEQ ID NO: 4 is characterized by a mutation of the amino acid sequence of human NGF (hNGF, SEQ ID NO: 2), wherein said mutation is associated with reduced nociceptive activity. In particular, arginine at position 100 of hNGF is substituted by glutamic acid. The present invention is based, in part, on the surprising finding that a therapeutic effect can be achieved without the side effects known from the prior art.

Without wishing to be bound to a particular theory, it is preferred that the polypeptide according to the present invention comprises one or more disulfide bridges, and most preferably three disulfide bridges. Mature and properly folded human NGF is characterized by three disulfide bridges (linking positions 136 ↔ 201, 179 ↔ 229, 189 - 231, position numbers refer to SEQ ID NO: 1; see Wiesmann et al., 1999, Nature, vol. 401, p. 184-188). Without wishing to be bound to a particular theory, it is preferred that the polypeptide according to the present invention comprises equivalent disulfide bridges (the position numbers of which are available to the skilled person by aligning the polypeptide according to the present invention with the polypeptide of SEQ ID NO: 1 and Wiesmann et al., supra.

### Description of presence and absence of adverse effects

Preferably, the treatment and/or prevention does not cause side effects or adverse effects in the subject to which the polypeptide is administered or has been administered. Thus, preferably, the administration of the polypeptide of the invention is not causative for any undesired effects in the mammalian subject.

One side effect or adverse effect that is preferably absent in this context is hyperalgesia or pain. It is specifically preferred that the treatment and/or prevention according to the present invention does not cause hyperalgesia in the mammalian subject. Thus, preferably, administration of the agent according to the present invention does not induce any hyperalgesic syndrome (pain).

It is important to point out that the absence of pain does not merely cause a more pleasant (or less unpleasant) treatment than the administration of a reference compound associated with pain (such as wild-type NGF), but is at least in part causative for the success of the treatment or prevention of eye disorders as such: considering that the polypeptide according to the present invention is preferably topically administered, more preferably topically administered onto the eye, the absence of pain will enable the treated subject to accept the administration of the polypeptide without adverse reactions such as scraping it off or washing it off or otherwise removing it in order to avoid to pain, and as a result of that, the polypeptide will exert is therapeutically beneficial effect, such as treatment or prevention of the eye disorder. Thus, the absence of pain associated with the polypeptide of the present invention will be suitable to overcome consumer reluctance and concerns of the regulatory authorities. In other words, the absence of pain is associated with a significant increase in the benefit-to-risk ratio compared to agents that are associated with pain.

In particular, preferably, the treatment and/or prevention does not cause hyperalgesia in the mammalian subject. In one embodiment, the subject to which the polypeptide of the invention is administered does not suffer from mechanic allodynia. More precisely, mechanic allodynia is not induced in the subject to which the polypeptide of the invention is administered, so that the subject to which the polypeptide is administered does not suffer from mechanic allodynia.

A further side effect or adverse effect that is preferably absent in this context is malignancy or cancer. In particular, the administration of the polypeptide of the present invention to a subject is preferably not associated with abnormal cell growth, and even more preferably is not associated with abnormal cell growth with the potential to invade or spread to other parts of the body. It is particularly preferred that the administration of the polypeptide of the present invention to a subject is not associated with cancer of the eye.

Thus, in summary, preferably, the administration of the polypeptide of the present invention to a subject is not associated with adverse effects such as malignancy and/or pain.

Typically, administration of the agent according to the present invention is well tolerated by the subject. In particular, preferably, administration of the polypeptide according to the present invention is not associated with the formation of anti-drug antibodies in the subject. Indeed, as the amino acid sequence of the polypeptide according to the present invention differs in only one or two amino acid positions from wild type human NGF, it is plausible that the immunological tolerability in humans is particularly advantageous, and it is plausible that administration of the polypeptide of the present invention is not associated with the formation of anti-drug antibodies in humans.

Preferably, administration according to the present invention positively influences one or more of the following: inflammation, extracellular matrix deposition, innervation and angiogenesis.

### Detectability of the polypeptide

Preferably, the polypeptide for use according to the present invention can be selectively recognized by a specific reagent with regard to endogenous (e.g. human) NGF. The terms "selectively recognized" and "detectable" are used interchangeably herein and generally refer to the specific identification, preferably by molecular means, of the protein, in a biological sample.

In that regard, the polypeptide according to the present invention is preferably detectable by an antibody or other immunoreactive molecule.

A protein detectable by an antibody or other immunoreactive molecule may also be referred to as an antigen. In some embodiments, a biological sample may be characterized by displaying - or not displaying - one or more specific antigens. In the context of the present invention, the polypeptide administered to the subject is preferably detectable in a biological sample obtained from the subject post administration of the polypeptide. One non-limiting way for showing presence of a protein is by Western Blot, but other immunological methods are equally comprised in the context of the present invention. The antibody or other immunoreactive molecule is either labelled (e.g. fluorophore-labelled) itself, or recognized by a labelled secondary antibody or other immunoreactive molecule, which is added for that purpose. Thus, in some cases, a secondary molecule that aids in the detection, such as e.g. an optionally labelled secondary antibody, is also added to facilitate detection.

According to the invention, an antigen is said to be present in a biological sample if the level is above the detection limit and/ or if the level is high enough to allow binding by antigen-specific antibodies added to the sample. According to the invention, an antigen is said to be not expressed on a cell if the level of expression is below the detection limit and/or if the level of expression is too low to allow binding by antigen-specific antibodies added to the sample.

An antibody or other immune reactive molecule may recognize an epitope on the cell. The term "epitope" refers to an antigenic determinant in a molecule such as an antigen, i.e., to a part in or fragment of the molecule that is recognized, i.e. bound, by the immune system, for example, that is recognized by an antibody or other immunoreactive molecule. Detection of an epitope specific for any particular antigen normally allows to conclude that that particular antigen is present on the cell being analyzed.

In one embodiment, a sample obtained from a subject, in particular the subject to which the polypeptide according to the present invention has been administered, can be characterized by immunophenotyping. "Immunophenotyping" generally means that the cell or sample can be characterized by antigen-specific molecules such as antibodies or other immune reactive molecules, which are added to the sample to determine if an antigen is present. Immunophenotyping includes cell sorting using various methods including flow cytometry, as well as analytic methods on lysed cells and lysed samples, such as Western Blotting.

In the present invention, a polypeptide that can be specifically detected even in the presence of wild-type NGF, such as wild-type human NGF, is particularly preferred. While any mutation of an amino acid sequence, such as any point mutation, for instance, may render a polypeptide specifically detectable even in the presence of the respective non-mutated wild-type polypeptide, and therefore each of the polypeptide of SEQ ID NO: 3 (not part of the present invention) and the polypeptide of SEQ ID NO: 4 may be prima facie specifically detected even in the presence of wild-type human NGF. It is particularly the polypeptide of SEQ ID NO: 4 for which an antibody is available that can distinguish said polypeptide from wild-type human NGF (WO 2008/006893 A1).

Thus, preferably the polypeptide is characterized by at least the absence of proline (which is present at position 61 of SEQ ID NO: 2, for reference) at position 61, more preferably by the substitution of proline at position 61 by another amino acid. In a particularly preferred embodiment, proline at position 61 is substituted by serine. The polypeptide for use according to the present invention is the polypeptide of SEQ ID NO: 4. This polypeptide is characterized by at least the absence of proline at position 61, more preferably by the substitution of proline at position 61 by another amino acid. In SEQ ID NO: 4, proline at position 61 of SEQ ID NO: 3 is substituted by serine.

### Ophthalmic disorder

According to the present invention, the polypeptide of SEQ ID NO: 4 is for the use in treatment and/or prevention of an ophthalmic disorder.

The ophthalmic disorder may be an inherited and/or an acquired ophthalmic disorder.

In some embodiments, the ophthalmic disorder is or comprises an eye lesion. In one embodiment the eye lesion comprises a damage and/or disorder of the optic nerve and/or retina.

Although terms like "ophthalmic disorder", "damage", "disorder", "eye", "optic nerve", "retina" and other terms are used in the singular form herein, the present invention is also applicable to subjects having multiple ophthalmic disorders, damages, disorders, and to administration to, and treatment of, all (both) eyes, all (both) optic nerves and all (both) retinas of the subjects.

Preferred disorders to be treated or prevented according to the present disclosure are diseases involved in the optic pathway. In general, optic pathway includes the retina, optic nerve, optic chiasm, optic radiations, and occipital cortex. Thus, preferred disorders to be treated or prevented according to the present disclosure are disorders affecting any one or more of the retina, optic nerve, optic chiasm, optic radiations, and occipital cortex. Damage along the optic pathway can cause a variety of visual field defects. Such visual field defects can be treated or prevented. Thus, the present disclosure also contemplates the treatment and/or prevention of visual field defects, including partial and complete vision loss.

Ophthalmic disorders include without limitation eyestrain, red eyes, night blindness, lazy eye, cross eyes (strabismus), nystagmus, color blindness, uveitis, presbyopia, blurred vision, eye pain, light sensitivity, floaters, dry eyes, excess tearing, cataracts, glaucoma, retinal disorders, conjunctivitis, corneal disorders, eye pain, eyelid problems, vision changes, reduced vision, problems associated with contact lenses, disorders associated with a damage and/or disorder of the optic nerve and disorders associated with a damage and/or disorder of retinal ganglion cells. The polypeptide according to the present invention may be administered to a subject suffering from a damage and/or disorder of the optic nerve and disorders associated with a damage and/or disorder of retinal ganglion cells, in order to treat the respective disorder. Alternatively or additionally, the polypeptide according to the present invention may be administered to a subject at risk of suffering from a damage and/or disorder of the optic nerve and disorders associated with a damage and/or disorder of retinal ganglion cells, in order to prevent the respective disorder. The present invention is also applicable, without limitation to the list of diseases including glaucoma, neurotrophic keratitis, optic neuritis, optic nerve atrophy, optic nerve head drusen and optic pathway glioma. Optic nerve neuropathies to which the present invention is applicable include without limitation the following: glaucoma, neurotrophic keratitis, optic neuritis, optic nerve atrophy, optic nerve head drusen and optic pathway glioma.

The polypeptide according to the present invention is for the use in treatment and/or prevention of an ophthalmic disorder that involves the optic nerve. In some embodiments, the optic nerve is damaged or affected. As shown e.g. in Example 4, the polypeptide of the present invention, when administered in an embodiment of the present invention, has a direct curative effect on such ophthalmic disorders. Thus, preferably, the ophthalmic disorder to be treated and/or prevented by the use of the polypeptide according to the present invention involves a damage and/or disorder of the optic nerve. Subjects having such damage and/or disorder of the optic nerve are described herein, and the present invention and following description of specific damages and/or disorders involving the optic nerve is applicable to all such subjects, unless the context dictates otherwise. Disorders involving a damage and/or disorder of the optic nerve include, without limitation, glaucoma, neurotrophic keratitis, optic neuritis, optic nerve atrophy optic nerve head drusen and optic pathway glioma. The present invention comprises the treatment and/or prevention of all these disorders, as well as of other disorders involving the optic nerve. In such embodiments, the agent according to the present invention is provided for use in the treatment of prevention of a disorder involving a damage and/or disorder of the optic nerve.

It is also described the use of the polypeptide in treatment and/or prevention of an ophthalmic disorder that involves the retina. In some embodiments, the retina is damaged or affected. The retina is the innermost, light-sensitive layer of tissue of the mammalian eye. The eye optically creates a focused two-dimensional image of the visual world on the retina, which translates that image into neural impulses to the brain to create visual perception. The neural retina consists of several layers of neurons interconnected by synapses and is supported by an outer layer of pigmented epithelial cells (photoreceptor cells). The ophthalmic disorder can be characterized by a disorder of the retina. For example, the disorder may be caused by a disorder of the neural retina and/or of the epithelial cells of the retina. Preferred retinal disorders include macular degeneration (age-related or not age-related), retinopathy (diabetic retinopathy or not), retinitis pigmentosa, retinoblastoma, cone-rod dystrophy (CORD, and retinal detachment (retinal separation), retinitis pigmentosa is normally inherited and leads to the loss of night vision and peripheral vision. Macular degeneration is characterized by loss of central vision because of death or impairment of the cells in the macula. In retinal separation, the retina detaches from the back of the eyeball. Hypertensive retinopathy and diabetic retinopathy are both characterized by damage to the tiny blood vessels that supply the retina and is in some embodiments caused by diabetes mellitus. Retinoblastoma is a cancer of the retina. In some cases, the optic nerve is physically damaged or affected, such as optic nerve crush (ONC, Example 4) and optic pathway glioma.

The present disclosure also describes the use of the polypeptide in a subject suffering from neuropathy. a subject may be suffering from neuropathy, such as in particular neuropathy of the optic nerve. Such subject may be diabetic or non-diabetic subjects. Neuropathy may be local or systemic. Reduction of neuropathy may be local and/or systemic. Reduction of neuropathy includes reduction of neuropathy in the eye. Optic nerve neuropathies include without limitation the following: glaucoma, optic pathway glioma, anterior ischemic optic neuropathy, post traumatic optic neuropathy, optic atrophy post hydrocephalus, trans-synaptic degeneration of the optic nerve fibers, compression of the pre-optic chiasmal pathway, autosomal dominant optic atrophy, Leber hereditary optic neuropathy, Wolfram syndrome optic neuropathy. Administration to children is explicitly contemplated in the invention, particularly but not limited to the following conditions: optic pathway glioma and optic atrophy post hydrocephalus.

The polypeptide according to the present invention is for the use in treatment and/or prevention of an ophthalmic disorder that involves retinal ganglion cells. In some embodiments, retinal ganglion cells are damaged or affected. Such diseases have been described e.g. by Garcia et al., 2016, Cytokin Groth Fact. Rev., vol. 34, p. 1359-1601 and by Levin et al., 2002, Progr. Retin. Eye Res., vol. 21, p. 465-484). In general, a retinal ganglion cell (RGC) is a type of neuron located near the inner surface (the ganglion cell layer) of the retina of the eye. However, all retinal ganglion cells also have a long axon that extends into the brain. These axons form the optic nerve, optic chiasm, and optic tract. Thus, many disorders involving the optic nerve are also diseases which involve retinal ganglion cells, and vice versa. These terms are not mutually exclusive. Equally, many disorders involving the retina are also diseases which involve retinal ganglion cells, and vice versa. These terms are not mutually exclusive. Disorders involving a damage and/or disorder of retinal ganglion cells include, without limitation, glaucoma, optic pathway glioma, anterior ischemic optic neuropathy, post traumatic optic neuropathy, optic atrophy post hydrocephalus, trans-synaptic degeneration of the optic nerve fibers, compression of the pre-optic chiasmal pathway, autosomal dominant optic atrophy, Leber hereditary optic neuropathy, Wolfram syndrome optic neuropathy. Administration to children is explicitly contemplated in the invention, particularly but not limited to the following conditions: optic pathway glioma and optic atrophy post hydrocephalus. In some embodiments, RCGs are physically damaged or affected, such as optic nerve crush (ONC, Example 4) and optic pathway glioma. The present invention comprises the treatment and/or prevention of all these and other disorders involving damage and/or disorder of RGCs. In such embodiments, the agent according to the present invention is provided for use in the treatment of prevention of a disorder involving a damage and/or disorder of RGCs.

The most preferred ophthalmic diseases to be treated and/or prevented according to the present invention are selected from the list consisting of glaucoma, neurotrophic keratitis, optic neuritis, optic nerve atrophy, optic nerve head drusen and optic pathway glioma.

### Subjects for which the agent according to the present invention is particularly suitable

According to the present invention, the polypeptide of SEQ ID NO: 3 (not part of the invention) or SEQ ID NO: 4 may be administered to a subject in need of such administration. A subject in need of such administration may be a subject suffering from a disorder described herein, a subject at risk of suffering from such a disorder, or otherwise afflicted with such a disorder. The agent is administered to the subject in a therapeutically effective amount. The therapeutically effective amount can be determined by the physician in view of the disclosure herein.

In particular, the polypeptide according to the invention is administered to a mammalian subject. The subject can also be referred to as "patient". Most preferably, the mammalian subject is a human.

The subject may be an adult subject or a non-adult subject, such as a child or an adolescent. Administration to children is explicitly contemplated in the invention.

The present invention also relates to a method of treating a patient suffering from an ophthalmic disorder, wherein the method comprises administering an effective amount of the polypeptide of SEQ ID NO: 3 (not part of the present invention) or the polypeptide of SEQ ID NO: 4 to the patient. The terms "patient" and "subject" are used interchangeably herein, particularly with reference to a patient/subject characterized by a ophthalmic disorder, as described herein.

The ophthalmic disorder is characterized by a damage and/or disorder of the optic nerve and/or of retinal ganglion cells of the subject. Such damage and/or disorder includes malfunction and reduced function of the retinal ganglion cells. Disorders characterized by a damage and/or disorder of the optic nerve and/or of retinal ganglion cells have been described e.g. above, and the following description of subjects is applicable to all such damages and/or disorders on such subjects, unless the context dictates otherwise.

In the context of the present invention, the term "prevent" is to be understood broadly and includes not only the prevention of onset of the disorder, but also the prevention of progression of the disorder. In particular, in the context of a disorder involving a damage and/or disorder of the optic nerve, the term "prevent also includes the prevention of further progression of the damage of the optic nerve.

In the context of the present invention, the term "treat" is to be understood broadly and includes without limitation the amelioration of the symptoms of the disorder. Indeed, it is preferred and also demonstrated by the experimental examples herein that achieving amelioration of the ophthalmic disorder, such as e.g. (partial) restoration of vision or other improvement or amelioration of the condition or disorder, is a preferred integral part of the invention as claimed herein. Indeed, attaining the claimed therapeutic effect is a functional technical feature of the present invention. The examples herein make plausible that said functional technical feature is achievable as a direct result of administration of the polypeptide of the present invention. In other words, the present inventors have identified that the polypeptide of the present invention is causative for achieving amelioration in a subject suffering from an ophthalmic disorder. The ophthalmic disorder is preferably characterized by a damage and/or disorder of the optic nerve.

The present invention is particularly suitable for a subgroup of subjects suffering from an ophthalmic disorder. Such subgroups are described herein. It is also possible that a particular subject falls into one or more of the subgroups described herein; administration of the polypeptide according to the present invention to subjects falling into one of the subgroups described herein is equally comprised by the present invention as administration of the polypeptide according to the present invention to subjects falling into more than one of the subgroups described herein.

The invention is not limited to particular causes of damage and/or disorder of the optic nerve. Mechanical causes of damage and/or disorder of the optic nerve are comprised in the invention as well as non-mechanical causes thereof. Further, diabetic causes of damage and/or disorder of the optic nerve are comprised in the invention as well as non-diabetic causes thereof.

In some embodiments, the polypeptide according to the present invention is optionally for administration to a subject who has undergone surgery. Accordingly, the polypeptide according to the present invention is suitable to treat or prevent the one or more postoperative complications in the eye. Alternatively, the polypeptide according to the present invention is also suitable to prevent surgery in a subject. For example, in optic pathway glioma, the optic nerve may be strengthened by administration of the polypeptide of the invention, and this may render surgery unnecessary or dispensable. Thus, the present invention also provides a non-invasive administration.

The present invention is suitable to treat ophthalmologic disorders, such as in particular damages and/or disorders of the optic nerve in diabetic and in non-diabetic subjects. Further details of the damages and/or disorders of the optic nerve are described hereinabove.

Ophthalmic disorders may occur in diabetic subjects. Such ophthalmic disorders can be treated and/or prevented based on the present invention.

In some embodiments, the mammal to which the polypeptide of the invention is administered, preferably a human, suffers from diabetes mellitus or has a predisposition to suffer from diabetes mellitus; a respective subject is referred to herein as "diabetic subj ect".

Diabetes mellitus is a common and debilitating disease that affects a variety of organs. Methods for detecting diabetes are well known in the art. Methods of detecting diabetes are, in one embodiment, not part of the present invention, but they may facilitate the treatment or prevention of an ophthalmic disorder in accordance with the present invention.

In typical embodiments the diabetes mellitus is selected among diabetes mellitus Type 1 and diabetes mellitus Type 2.

Optionally, the ophthalmic disorder comprises a disorder caused by diabetes mellitus or otherwise involving diabetes mellitus. Thus, the present invention is applicable to subjects suffering from diabetes mellitus as well as to subjects not suffering from diabetes mellitus. In some embodiments, the agent according to the present invention is for administration to a diabetic subject. Thus, the polypeptide according to the invention may be administered to the eye in a diabetic subject. Thus, in one embodiment, the use of the polypeptide according to the present invention comprises administration to the eye of a diabetic subject.

Thus, the present invention provides a treatment and/or prevention to ophthalmic conditions in a subject affected by diabetes. Indeed, according to the present invention, an effective medical treatment may not only help the patients recover from these eye complications, but may also lead them to a better quality of life and a reduction of medical care and/or expense.

Thus, the present invention provides an advantage over current treatment methods, which oftentimes may not be able to provide an effective method to treat ophthalmic disorders. The present invention provides a treatment and/or prevention of such ophthalmic disorders.

### Administration to the Eye

Treatment or prevention according to the present invention may be carried out by administration, preferably topical administration, of the polypeptide of the invention. According to the present invention, the eye of the subject is the preferential site of administration of the polypeptide of the invention. In general, when it is said herein that the polypeptide is administered onto the eye of a subject, such administration may be either onto the surface of the eye, or into the eye, unless the context dictates otherwise.

Preferably, the polypeptide is for administration to the eye. More preferably, administration is selected from the group consisting of topical administration to the eye and intravitreal administration, whereby topical administration to the eye is most preferred.

In preferred embodiments, the agent according to the present invention is for use in treatment or prevention of a disorder involving a damage and/or disorder of the optic nerve, and for that purpose, is administered to the eye. According to the present invention, the polypeptide is suitable for the treatment or prevention of damages and/or disorders of the optic nerve, and for such purposes, is administered to the eye.

The present invention is not limited to subjects having an ophthalmic disorder in one eye only, nor to those having an ophthalmic disorder in both eyes. Thus, the terms "eye" and "optic nerve", independent of their use in the singular or plural form in the present disclosure, are explicitly inclusive of all those singular and plural embodiments.

In some embodiments, the administration is carried out at a hospital. In some embodiments, the treatment is not carried out a hospital. For example, an eye drop administration will normally not require hospitalization of the subject.

Optionally but not mutually exclusive the ophthalmic disorder comprises at least one mechanical injury. Thus, the present invention also comprises the treatment or prevention of mechanical injuries, whereby treatment of such injuries is practically more meaningful than prevention. Such disorders include those involving optic nerve crush (ONC) and other disorders affecting the optic nerve.

In summary, and as detailed herein, according to the present invention, the polypeptide is administered to the eye of a subject suffering from or at risk to suffer from an ophthalmic disorder.

In another embodiment the agent according to the present invention is for the treatment or prevention of cancers on the eye, such as without limitation optic pathway glioma, and/or of eye disorders associated with such cancers.

In a further embodiment the agent according to the present invention is for the treatment or prevention of an ophthalmic disorder which results from a genetic disorder in the subject or is influenced by a genetic disorder in the subject.

### Route of Administration

The present invention provides a heterologous polypeptide for administration to a subj ect.

Preferably, the polypeptide is for topical administration. Thus, preferably, the polypeptide of the invention is administered to the eye. In some embodiments, the polypeptide is administered intravitreally. In some embodiments, the polypeptide is administered topically to the eye.

More preferably, the polypeptide is administered onto the surface of the eye. In other words, the polypeptide according to the present invention is preferably topically administered, more preferably topically administered onto the eye. Most preferably, the polypeptide is administered onto the conjunctiva of the subject. Administration onto the conjunctiva of the subject is also termed "conjunctival" administration. Conjunctival administration is most preferably carried out by administering eye drops.

The administration according to the present invention typically does not involve surgery of the subject. In one embodiment, administration of the polypeptide of the invention does not comprise or encompass an invasive step representing a substantial physical intervention on the body which requires professional medical expertise to be carried out and which entails a substantial health risk even when carried out with the required professional care and expertise. In contrast, in more typical embodiments, administration of the polypeptide of the invention, particular the topical administration, is generally considered safe for the subject and therefore the polypeptide may be administered by the subject himself or herself, particularly in case of a human subject.

Optionally, the eye is covered by a plaster and/or eye dressing prior to and/or during and/or after the administration. The enormous variety of types of plasters and/or eye dressings available is not limited by the present invention. Thus, any plasters and/or eye dressing may be used unless technically clearly inappropriate. Plasters and/or eye dressings suitable for covering the eye are preferred. In some embodiments, the polypeptide according to the invention is administered simultaneously to application of a plaster or eye dressing; optionally, the plaster or eye dressing comprises the polypeptide of the invention, optionally in the form of an aqueous medium applied to the eye dressing prior to administration.

In an alternative and more preferred embodiment, the polypeptide is administered repeatedly. In a particularly preferred embodiment, the polypeptide is administered repeatedly one to five times per day. In one embodiment, the polypeptide is administered one time per day. In one embodiment, the polypeptide is administered two times per day. In one embodiment, the polypeptide is administered three times per day (see also Example 4). In one embodiment, the polypeptide is administered four times per day. In one embodiment, the polypeptide is administered five times per day. It is particularly preferred that the polypeptide is administered to a human subject twice per day. All aforementioned administrations are preferably repeated over a course of several days, as disclosed herein. For example, the polypeptide may be administered repeatedly for a period of three to 30 days, preferably seven to 14 days, and preferably one to five times per each of these days.

Preferably, the agent according to the present invention, when administered to the eye as described herein, does not cause a hyperalgesic syndrome (pain). Thus, the agent according to the present invention may come into contact with nociceptive fibers (nerves), including the optic nerve, without causing a hyperalgesic syndrome (pain). For this and other reasons, the present invention provides a major advantage, particularly for the treatment and/or prevention of disorders involving the optic nerve.

### Timing of administration

In general, the polypeptide according to the present invention is administered repeatedly or in a single administration.

In one embodiment, the polypeptide is administered in a single administration. In that embodiment, the polypeptide is administered in a single administration, and administration is discontinued after that single administration.

In an alternative embodiment, the polypeptide is administered repeatedly for a period of three to 30 days, preferably seven to 14 days. Optionally, administration is discontinued after completion of said interval.

In one embodiment, the polypeptide is administered repeatedly. In one embodiment, the polypeptide is administered repeatedly, e.g. until complete healing of the ophthalmic disorder, or at least until melioration of the symptoms of the disorder is observed. Alternatively, the polypeptide is administered repeatedly for a period of three to 30 days, preferably seven to 14 days. Optionally, administration is discontinued after completion of said interval. In a particularly preferred embodiment, the polypeptide is administered repeatedly at least three times per day.

Preferably, the polypeptide is administered following a damage of the optic nerve.

Preferably, the polypeptide is administered as soon as possible after the diagnosis of optic nerve damage in order to minimize the extent of RGCs' death. "as soon as possible" includes embodiments of one day or less after the diagnosis of optic nerve damage. However, the polypeptide is still neuroprotective if administered days after the occurrence of damage of the optic nerve. Preferably, the polypeptide is administered at least three days after induction of a damage of the optic nerve. Preferably, the polypeptide is administered at least four days after induction of a damage of the optic nerve. Preferably, the polypeptide is administered at least five days after induction of a damage of the optic nerve. Preferably, the polypeptide is administered at least six days after induction of a damage of the optic nerve. Preferably, the polypeptide is administered at least seven days after induction of a damage of the optic nerve. Preferably, the polypeptide is administered at least eight days after induction of a damage of the optic nerve. Preferably, the polypeptide is administered at least nine days after induction of a damage of the optic nerve. Preferably, the polypeptide is administered at least ten days after induction of a damage of the optic nerve. Most preferably, however, the polypeptide is administered at least four days after induction of a damage of the optic nerve. In all these foregoing embodiments, "at least (number) days after induction of damage" is intended to mean, in the case of repeated administration, that the first dose is administered after the indicated number of days. Optionally, further doses may be administered later, on the same day and/or on subsequent days, in line with the disclosure herein.

### Dose

The agents and compositions described herein are administered in effective amounts. According to the present invention, an "effective amount" is the amount or dose which achieves a desired reaction or a desired effect, either alone or together with further doses. In the case of treatment of a particular disorder, the desired reaction preferably relates to inhibition of the course of the disease. This comprises slowing down the progress of the disease and, preferably, interrupting or reversing the progress of the disease. The desired reaction in a treatment of a disease or of a condition may also comprise a delay of the onset or a prevention of the onset of said disease or said condition. In some embodiments the desired reaction comprises the complete healing of the symptoms of the disorder, locally and/or systemically.

An effective amount of an agent or composition described herein will depend on the condition or disorder to be treated, the severity of the disorder, the individual parameters of the subject to which the agent is administered, such as age, physiological condition, accompanying condition(s) (if present), size and weight, the duration of treatment, the type of an accompanying therapy (if present), the specific route of administration and other parameters. Accordingly, the doses administered of the agents described herein may depend on various of such parameters. In the case that a reaction in a patient is insufficient with an initial dose, higher doses (or effectively higher doses achieved by a different, more localized route of administration) may be used.

According to the present invention, suitable and therapeutically effective dosages for the administration of a therapeutic agent for administration to a human subject for the treatment and/or prevention of an eye can be determined based on experimentally determined suitable and therapeutically effective dosages for the administration of a therapeutic agent for administration to a rodent subject, particularly, a mouse, for the treatment and/or prevention of an eye disorder.

Animal models (Example 4) are helpful in establishing pharmacological responses, as well as assessing potential toxicities of treatment products. In some embodiments the dose to be administered to the subject is a dose as disclosed in Example 4 or in Example 5 or in Example 6.

Preferably, the dose of the polypeptide is determined at or before the onset of treating. In one embodiment, the dosing is adjusted for later administration(s), depending on the progression of the treatment. In an alternative embodiment, the dosing is not adjusted for later administration(s), so that subsequent dosages correspond to the first dose.

The polypeptide is active both via topical (e.g.) conjunctival and via intravitreal administration. Specifically for topical (most preferably conjunctival) administration (preferably eyedrops) the preferred dose/each dose has an amount of 0.3 to 30 µg of the polypeptide per eye, more preferably 1 to 10 µg of the polypeptide per eye, and most preferably about 5 µg of the polypeptide per eye. Most preferably, these indicated dosages are specifically for administration to the human eye.

### Process for obtaining the polypeptide

In one embodiment, the polypeptide of SEQ ID NO: 3 (which is not part of the present invention) and the polypeptide of SEQ ID NO: 4 is obtainable from a biological source. Optionally, the polypeptide of SEQ ID NO: 3 (which is not part of the present invention) and the polypeptide of SEQ ID NO: 4 is obtainable by recombinant expression. For that purpose, and open reading frame encoding the respective polypeptide is introduced into a source of recombinant proteins, for example a host cell or a cell-free system for protein expression. Indeed, considering that human NGF is produced only in minute quantities *in vivo,* mouse NGF is usually produced as a heterogeneous mixture of various proteins (see WO 2000/022119 A1), and the polypeptides of the present invention are non-natural and thus not produced *in vivo* at all, the most meaningful possibility to produce the polypeptide of the present invention is by recombinant expression, in accordance to equivalent suggestions for wild-type NGF in the state of the art (WO 2000/022119 A1, WO 2008/006893 A1; Rattenholl et al., Eur. J. Biochem, 2001, vol. 268, p. 3296-3303, US 2018/0086805 A1). However, it has been a constant challenge to obtain such polypeptides at a purity grade sufficient for administration to a mammal. This challenge has been overcome by the present invention, as disclosed in detail herein (see also Examples 1 and 2).

Preferably, the polypeptide according to the present invention is obtainable by recombinant expression in bacteria. More preferably, the polypeptide according to the present invention is obtainable by cytosolic recombinant expression in bacteria. In general, bacterial cells, in particular *E. coli,* are capable of recombinant production of high amounts of recombinant proteins, but, as is the case for many other recombinantly expressed genes, the production of recombinant NGF and similar polypeptides in bacteria results in a biologically inactive translation product which is then accumulated in the cell (cytosol) in the form of aggregates (so-called inclusion bodies (IBs) (WO 2000/022119 A1; US 2018/0086805 A1). In contrast to NGF, pro-NGF is known to be rather unstable and requires high efforts for refolding and purification at low recovery rates, which renders the process of NGF production via pro-NGF in bacteria relatively difficult and expensive. Thus, the main difficulties associated with bacteria-produced NGF and similar bacteria-produced polypeptides, via the respective pro-forms, concern the folding, the processing and the purification of the recombinant protein. These difficulties have now been solved (see Example 1 and 2). As a result, the polypeptides of SE ID NO: 3 (not part of the invention) and SEQ ID NO: 4 become available at a purity grade suitable for administration to a mammal, including a human.

Preferably, the polypeptide according to the present invention is expressed together with a pro-sequence. Without limitation, a suitable pro-sequence is the pro-sequence of wild-type human NGF (amino acid positions 18 to 121 of SEQ ID NO: 1), typically fused to the N-terminus of the polypeptide of SEQ ID NO: 3 or 4. For wild-type NGF, although not being part of mature NGF, and hence not required for the biological function of NGF, the presence of the covalently attached pro-sequence was shown to promote re-folding of recombinant NGF from inclusion bodies with concomitant disulfide bond formation of the mature part (beta-NGF). Thus, the presence of the covalently attached pro-sequence positively influences the yield and rate of re-folding when compared to the *in vitro* re-folding of mature NGF from inclusion bodies (Rattenholl et al., Eur. J. Biochem, 2001, vol. 268, p. 3296-3303). Without wishing to be bound to a particular theory, the same is plausible and postulated herein for the polypeptide of SEQ ID NO: 3 (not part of the invention) and SEQ ID NO:4.

Thus, when polypeptide according to the present invention has been produced in inclusion bodies, correct folding is required, and this is normally achieved post-translationally, as is the cleavage from the covalently attached pro-sequence; sophisticated methods for folding, cleavage and purification have been proposed in the past, in particular for wild-type human NGF. Notably, most of published studies on NGF apply a general refolding regime which was previously established by Rattenholl et al. (2001, Eur. J. Biochem, vol. 268, p. 3296-3303). Within this original study, several parameters of protein refolding (e.g. temperature, refolding time, pH of refolding reaction, arginine, glutathione and protein concentration) were investigated in detail and their effect on the refolding efficiency was assessed. The protocol by Rattenholl et al. relies on the re-naturation of the pro-form, which has a very poor solubility, obtainable from inclusion bodies after recombinant production in prokaryotes, whereby pro-NGF is solubilized in a solution of a denaturing agent in a denaturing concentration, transferred into a solution which is not or weakly denaturing, so that the solubility is maintained and the dissolved denatured pro-NGF can assume a biologically active conformation, including formation of disulfide bonds as in native NGF, and afterwards the NGF is purified and the pro-sequence is removed proteolytically (WO 2000/022119 A1; Rattenholl et al., Eur. J. Biochem, 2001, vol. 268, p. 3296-3303). Notably, within this study it was found that a low protein concentration leads to a higher specific yield of correctly folded product as compared to a higher protein concentration. Exemplary, protein concentrations around 50 mg per liter of refolding reaction resulted in a specific yield of ~ 25 % correctly folded pro NGF, while this fraction was reduced to 10 % at protein concentrations of 500 mg per liter. Based on that, Rattenholl et al. suggest that the protein-concentration in the refolding solution has to be very low: according to Rattenholl et al., 15-20 mg of correctly folded protein per liter of refolding reaction are expected as yield. However, this would require a scale-up (e.g. beyond laboratory scale) for purification of even a few hundred mg recombinant protein.

While human pro-NGF contains a native cleavage site for the protease Furin (Arg¹-Ser²-Lys³-Arg⁴; R¹S²K³R⁴), and Furin cleaves pro-NGF at that site *in vivo,* Furin is not available at commercially relevant purity or quantity. According to the present invention, the polypeptide according to the present invention, when expressed together with a prosequence, e.g. in *E. coli,* is preferably cleaved by the protease Trypsin (EC 3.4.21.4), which is available commercially. Indeed, for wild-type NGF it has been reported that Trypsin would yield satisfying biologically active, mature NGF, which can be eventually purified (Rattenholl et al., Eur. J. Biochem, 2001, vol. 268, p. 3296-3303), and Trypsin-based proteolysis of recombinantly expressed pro-NGF has meanwhile been adopted by others (e.g. D'Onofrio et al., 2011, PLoS One, vol. 6, e20839). However, it was later shown that cleavage of the wild-type pro-NGF with trypsin to produce beta-NGF is associated with several drawbacks, as low amounts of trypsin would lead to inefficient cleavage, whereas high amounts of trypsin would further decrease the selectivity of the cleavage, as trypsin is capable of cleaving C-terminally of any arginine and lysine residue (R and K residue), so that by digestion of R¹S²K³R⁴-containing pro-NGF by trypsin, several alternative digestion products would be obtained; thereby the use of trypsin as cleavage enzyme would lead to very low yields of correctly cleaved NGF, and to purification and yield problems, as the different cleavage products are not economically separated under standard conditions. As one solution, it was proposed to express a variant of pro-NGF, wherein the protease cleavage site R¹S²K³R⁴ in the pro-peptide is substituted at least at positions R¹ and K³ corresponding to positions 101 and 103 of the human wildtype pro-NGF sequence (SEQ ID NO: 1) by another amino acid (WO 2013/092776 A1). In one example, R¹ and K³, respectively, are replaced by valine (V) and alanine (A), transforming the original Furin cleavage site R¹S²K³R⁴ into V¹S²A³R⁴, wherein Trypsin is capable of cleaving specifically only C-terminally of R⁴; Trypsin-mediated cleavage of a respective pro-NGF can also be referred to as the "VSAR method". Although WO 2013/092776 A1 is silent on then polypeptides of SEQ ID NO: 3 or 4 according to the present invention, the VSAR method has been initially proposed to be applicable to certain variants of pro-NGF muteins, although it was reported that the proteolysis conditions needed to be titrated with care (US 2018/0086805 A1). In the course of arriving at the present invention, the present inventors found that the VSAR technology, contrary to earlier suggestions, does not satisfactorily solve purity issues associated with the recombinant production of the polypeptide of the present invention at satisfactory purity. Indeed, the purification of recombinantly expressed beta-NGF or muteins thereof, not only from host cell proteins (HCP), but also from trypsin (or other protease used for cleavage) is still a challenge; needless to say, it would be required that a proteolytic enzyme (such as trypsin) be absent from a final preparation of a pharmaceutical protein, in order to avoid proteolysis during storage of the polypeptide, so that the polypeptide is substantially pure and un-degraded at the time point of administering it to a subject, according to the present invention. The present inventors have solved this challenge, as is described herein. Thus, the polypeptides according to SEQ ID NO: 3 (not part of invention) or SEQ ID NO:4 are made available at high purity and thus essentially free of trypsin and/or of degradation products of the polypeptide. Although certain methods for the production of NGF (e.g. WO2013092776 A1) and of the polypeptide of SEQ ID NO: 4 (e.g. Malerba et al., 2015, PLOS One, vol. 10, e0136425) have been previously described, the present disclosure shows that surprisingly, previously published processes are insufficient for obtaining the respective polypeptide at high purity. As a solution to these insufficiencies, the present disclosure provides a new process and related aspects, as described in detail herein.

A process for obtaining the polypeptide of SEQ ID NO: 3 (not part of the invention) and the polypeptide of SEQ ID NO: 4 from recombinant expression, e.g. in a host cell, may comprise purification. Purification, in the broadest sense, means that the polypeptide of SEQ ID NO: 3 (not part of the invention) or SEQ ID NO: 4 is separated from other molecules, including other proteins, such as host cell proteins. Thus, purification may include separation from one or more other molecules, including other proteins, such as host cell proteins, proteases (e.g. trypsin) and/or degradation products of the polypeptide according to the invention.

The process for production of the polypeptide of SEQ ID NO: 3 (not part of the invention) and the polypeptide of SEQ ID NO: 4 according to the present invention preferably comprises the following steps:
(a) obtaining a precursor of the polypeptide of SEQ ID NO: 3 or SEQ ID NO: 4,
(d) purification,
and the purification in step (d) typically comprises purification on a mixed mode stationary phase. Thus, in one embodiment, the polypeptide of SEQ ID NO: 3 (not part of the invention) or SEQ ID NO: 4 is obtainable by recombinant expression and purification, wherein the purification comprises purification on a mixed mode stationary phase. The term "on mixed mode stationary phase" is to be understood broadly and means that a mixture comprising the polypeptide of SEQ ID NO: 3 (not part of the invention) or SEQ ID NO: 4 or precursor of any of these, together with other molecular species, is exposed to a mixed mode stationary phase, e.g. by chromatography or other suitable process step. Indeed, preferably a mixture comprising the polypeptide of SEQ ID NO: 3 (not part of the invention) or SEQ ID NO: 4 or precursor of any of these, together with other molecular species, is subjected to chromatography, so that the purification in step (d) comprises purification by mixed mode chromatography. Preferably, the mixed mode chromatography comprises the use of a stationary phase having a charged group, preferably negatively charged group, and an aromatic group and/or a hydrophobic group.

Purification, in the broadest sense, according to the present invention, means that the polypeptide of SEQ ID NO: 3 (not part of the invention) or SEQ ID NO: 4 thereof is at least partially separated from other molecular species, including other proteins, such as host cell proteins, precursor and/or degradation products. As a result, the polypeptide of SEQ ID NO: 3 (not part of the invention) or SEQ ID NO: 4 which is at least partially purified is obtainable. While the other molecular species may be optionally discarded or not, the polypeptide of SEQ ID NO: 3 (not part of the invention) or SEQ ID NO: 4 is preferably obtained and retained as a result of the purification.

Preferably, the mixed mode chromatography comprises the use of a stationary phase having a charged group, preferably negatively charged group, and an aromatic group and/or a hydrophobic group.

Each of these steps may itself comprise several actions, which, for simplicity, can also be referred to as steps. For illustration, and as detailed below, step (d) may comprise more than one purification step, e.g. on more than one stationary phase.

Any letter or number used herein in relation to one or more process steps, such as e.g. (a), (b), (c), (d), (d1), (d2), is not to be understood as limiting, but rather for reference. It should not be understood that the sequence of events in the process or use according to the present invention may be limited by alphabetical sequence of letters or the numerical sequence of numbers. Notwithstanding the foregoing, it is strongly preferred that the sequence of events in the process or use according to the present invention is a sequence described herein.

Additional aspects of the mixed mode chromatography, particularly suitable stationary phases, will be described in some more detail below, but these aspects are generally applicable to the present invention. Thus, in particular all those stationary phases, including all embodiments thereof, that are described below to be particularly useful for the mixed mode chromatography in step (d2) are generally useful for the purification of the polypeptide of SEQ ID NO: 3 (not part of the invention) and/or the polypeptide of SEQ ID NO: 4 according to the present invention, and can be used in all types of embodiments, such as in combination with a step of (d1) capturing chromatography or without. Indeed, Example 2B describes that some advantages can be achieved by using mixed mode chromatography in a variation of a protocol according to the state of the art.

Optionally, the polypeptide of SEQ ID NO: 3 (not part of the invention) or the polypeptide of SEQ ID NO: 4 is obtainable in a process which comprises (re-)folding and/or chromatographic purification and/or protease digestion, and optionally adjustment to final protein concentration and/ preparation of a desired formulation.

Thus, the administration of the polypeptide of SEQ ID NO: 3 (not part of the invention) or SEQ ID NO: 4 to a subject in need thereof as disclosed herein is also enabled through the industrially relevant purity and yield of the polypeptide of SEQ ID NO: 3 or SEQ ID NO: 4, which is available to the skilled person based on the disclosure herein. Thus, the present disclosure also describes a process for production of the polypeptide of SEQ ID NO: 3 (not part of the invention) or SEQ ID NO: 4.

The process for production of the polypeptide of SEQ ID NO: 3 (not part of the invention) or SEQ ID NO: 4 according to the present invention preferably comprises the following steps:
(a) obtaining a precursor of the polypeptide of SEQ ID NO: 3 or SEQ ID NO: 4, e.g. by recombinant expression,
(d) purification, wherein the purification comprises purification on a mixed mode stationary phase.

It is also preferred in the present invention that the precursor of the polypeptide of SEQ ID NO: 3 (not part of the invention) or SEQ ID NO: 4 is subjected to a step
(c) exposure to a protease.

Said exposure is typically carried out prior to step (d).

The process of the present invention is preferably also characterized in that no chromatographic purification is performed prior to the exposure to protease. Indeed, the present disclosure (Examples 1, 2) shows that the digestion with protease works well and efficient also in a crude fraction obtained from a host cell, i.e. when no chromatographic purification has been performed prior to the exposure to protease.

Preferably, the step of obtaining (a) comprises expression of a precursor of the polypeptide of SEQ ID NO: 3 (not part of the invention) or SEQ ID NO: 4, preferably recombinant expression. More preferably the recombinant expression is in a host cell. After culturing the host cell, the polypeptide of SEQ ID NO: 3 (not part of the invention) or SEQ ID NO: 4 is obtained in a fraction of the cell culture. The fraction may consist of the host cells, i.e. in case the protein is substantially not secreted from the host cells. This is the case e.g. when the polypeptide of SEQ ID NO: 3 (not part of the invention) or SEQ ID NO: 4 is produced in inclusion bodies and/or otherwise in an intracellular compartment including the cytosol. Suitable host cells can be selected from prokaryotic and eukaryotic host cells, although prokaryotic host cells are preferred in typical embodiments. Preferred prokaryotic host cells include *Escherichia coli (E. coli),* preferably *E. coli* Rosetta (DE3). In one embodiment, the polypeptide of SEQ ID NO: 3 (not part of the invention) or SEQ ID NO: 4 is obtained in a conformation other than the native conformation and/or in aggregates, most preferably in inclusion bodies. Then, preferably the process of the present invention comprises a step (b) of (re-)folding the polypeptide of SEQ ID NO: 3 (not part of the invention) or SEQ ID NO: 4. Preferably, step (c) is carried out after step (b).

Preferably, in step (c) the protease is a protease capable of cleaving the polypeptide of SEQ ID NO: 3 (which is not part of the invention) or SEQ ID NO: 4 in such a manner that the (mature) polypeptide of SEQ ID NO: 3 or SEQ ID NO: 4 is released. In a particular embodiment, said protease is trypsin, preferably porcine trypsin, optionally recombinantly expressed.

Preferably, the step of purification (d) comprises the following steps, preferably in sequential order:
(d1) capturing,
(d2) polishing.

Preferably, the step of capturing (d1) is carried out by chromatography, preferably column chromatography. More preferably, said step of capturing (d1) is carried out using a cation exchange chromatography stationary phase or a mixed mode chromatography stationary phase. Even more preferably said step of capturing (d1) is carried out using a mixed mode chromatography stationary phase, which is preferably Capto MMC.

Preferably, the step of polishing (d2) is carried out by chromatography, preferably column chromatography. More preferably, said step of polishing is carried out using a cation exchange chromatography stationary phase. Even more preferably said step of capturing (d1) is carried out using SP sepharose, preferably SP sepharose with a small particle size. SP is an abbreviation for sulfopropyl.

Optionally, the process according to the present invention comprises an additional step of adjustment to final protein concentration and/ preparation of a desired formulation. As a result, a composition according to the invention is obtainable.

In other terms, the present invention provides mixed mode chromatography for the preparation of the polypeptide of SEQ ID NO: 3 (which is not part of the invention) or SEQ ID NO: 4. The mixed mode chromatography is useful in the preparation of the polypeptides. In preferred embodiments, the precursor of the polypeptide according to the invention is exposed to a protease for the purpose of digestion, and the mixed mode chromatography is used in a step subsequent to exposure to the protease. In preferred embodiments, no chromatographic purification of the polypeptide of the invention is performed prior to said exposure to protease.

### Purity of the polypeptide

The polypeptide of the invention is substantially or essentially free from components that normally accompany it in its native state. The polypeptide of the invention is isolated before being administered. In one embodiment, the "isolated polypeptide" refers to the polypeptide, which has been purified from the cellular and extracellular environment, such as tissue, which surround it in a naturally-occurring state, e.g., from the cell in which it has been expressed, such as a host cell. In another embodiment, "isolated polypeptide" refers to *in vitro* isolation and/or purification of a polypeptide, respectively, from its natural cellular environment, and from association with other components of the environment in which the polypeptide normally resides.

Preferably, the polypeptide of SEQ ID NO: 3 (which is not part of the invention) or SEQ ID NO: 4 for use according to the present invention is substantially free of impurities. Such advantageously pure polypeptide of SEQ ID NO: 4 is obtainable as described herein.

The polypeptide according to the invention is regarded as a pharmaceutically active peptide or protein.

In a particularly advantageous embodiment of the present invention the polypeptide according to the present invention is obtained essentially free of degradation products of said polypeptide. In particular, the present disclosure shows that, contrary to reports on wild type human NGF in the state of the art, the exposure of a precursor of SEQ ID NO: 4 to trypsin will inherently partially cleave said precursor C terminally of arginine (Arg, R) residue 9 of SEQ ID NO: 4, either before or after purification, if purification does not completely remove trypsin (des-nona variant, data not shown). By the specific method of purification as provided in the present invention, the polypeptide according to the present invention can be obtained essentially free of trypsin and/or of the des-nona variant.

Preferably, the polypeptide obtainable as described above is essentially free of degradants of the polypeptide. In particular, the present disclosure makes the polypeptide of the invention available at a new, improved purity grade, and it is preferred that the polypeptide is administered at such high purity. Preferably, the polypeptide of the invention for use according to the invention is characterized by a purity grade of at least 90 %. More preferably, the polypeptide of the invention for use according to the invention is characterized by a purity grade of at least 91 %. More preferably, the polypeptide of the invention for use according to the invention is characterized by a purity grade of at least 92 %. More preferably, the polypeptide of the invention for use according to the invention is characterized by a purity grade of at least 93 %. More preferably, the polypeptide of the invention for use according to the invention is characterized by a purity grade of at least 94 %. More preferably, the polypeptide of the invention for use according to the invention is characterized by a purity grade of at least 95 %. More preferably, the polypeptide of the invention for use according to the invention is characterized by a purity grade of at least 96 %. More preferably, the polypeptide of the invention for use according to the invention is characterized by a purity grade of at least 97 %. More preferably, the polypeptide of the invention for use according to the invention is characterized by a purity grade of at least 98 %. Even more preferably, the polypeptide of the invention for use according to the invention is characterized by a purity grade of at least 99 %.

Most preferably, the polypeptide of the invention for use according to the invention is characterized by a purity grade of more than 99.0 %, such as a purity grade of more than 99.1 %, more than 99.2 %, more than 99.3 %, more than 99.4 % %, more than 99.5 %, more than 99.6 %, more than 99.7 %, more than 99.8 % %, more than 99.9 %.

Herein, "purity grade" generally refers to the weight (w) percentage of the polypeptide according to the present invention with respect to the weight (w) of biological material other than the polypeptide of the present invention. For illustration, in general, at a purity grade of 99.0 %, the polypeptide of the present invention is present at a relative amount (weight) of 99.0 units (e.g. 1.0 mg), and the sum of the weight of all biological material other than the polypeptide of the present invention is 1.0 units (e.g. 1.0 mg). Such biological material other than the polypeptide of the present invention includes, without limitation, host cell proteins, nucleic acids, protease(s) such as e.g. trypsin, inactivated or not, degradation products of the polypeptide of the invention such as and other macromolecules of biological origin. In a particular embodiment, the "purity" grade refers to the purity vs. polypeptides other than polypeptides of the invention. For illustration, in that embodiment, at a purity grade of 99.0 %, the polypeptide of the present invention is present at a relative amount (weight) of 99.0 units (e.g. 1.0 mg), and the sum of the weight of all polypeptides which are nonidentical to the polypeptide of the present invention is 1.0 units (e.g. 1.0 mg). Degradation products of the polypeptide of the present invention, for the avoidance of doubt, are included in the "polypeptides which are nonidentical to the polypeptide of the present invention". A particular degradation product is the des-nona variant (see Examples 1 and 2).

In particular essentially free of the des-nona variant of the polypeptide. The des-nona variant is a previously uncharacterized degradation product of the polypeptide of the present invention that is associated with production of certain variants of NGF including the polypeptide of the present invention, unless the polypeptide is produced by the new method disclosed herein (see e.g. Examples 1 and 2). "essentially free" in this context is intended to mean that the polypeptide of the invention for use according to the invention is characterized by a purity grade, with respect to the des-nona variant, of more than 99.0 %, such as a purity grade of more than 99.1 %, more than 99.2 %, more than 99.3 %, more than 99.4 % %, more than 99.5 %, more than 99.6 %, more than 99.7 %, more than 99.8 % %, more than 99.9 %, all with respect to the des-nona variant. In the most preferred embodiment, the des-nona variant is undetectable and/or absent.

It is also preferred that the polypeptide according to the present invention is essentially free of any protease (such as trypsin). "essentially free" in this context is intended to mean that the polypeptide of the invention for use according to the invention is characterized by a purity grade, with respect to the sum of all proteases (including trypsin), of more than 99.0 %, such as a purity grade of more than 99.1 %, more than 99.2 %, more than 99.3 %, more than 99.4 % %, more than 99.5 %, more than 99.6 %, more than 99.7 %, more than 99.8 % %, more than 99.9 %, all with respect to the sum of all proteases (including trypsin). In the most preferred embodiment, trypsin is undetectable and/or absent.

Such high purity grade, in the above-described embodiments, is associated with improved acceptability by regulatory authorities and qualifies the polypeptide of the present invention as a medicament for use in mammalian subjects, including humans, in particular. Thus, the purity grade according to the present invention enables for the first time the use of this polypeptide for administration to the eye, including the human eye, in a safe and reliable manner. The high purity grade with respect to protease (trypsin) in particular enables storage of the polypeptide also in non-frozen form.

### Compositions

In the following, compositions comprising the polypeptide of the present invention will be described.

In the compositions according to the present invention, the polypeptide of SEQ ID NO: 3 (which is not part of the invention) or SEQ ID NO: 4 is comprised as active ingredient. Further ingredients may be comprised.

In one embodiment, the polypeptide is comprised in an aqueous medium. Said aqueous medium is for administration to the mammalian subject.

A particular aqueous composition for use according to the present invention is a liquid composition suitable for use as eye drops. In general, eye drops are drops of a liquid which are suitable to administer on the ocular route. The term "eye drops" is not particularly limited, and generally refers to a composition, typically an aqueous liquid composition, that can be administered to the eye without causing damage to the eye. In general, eye drops have less of a risk of side effects than e.g. oral medicines or medicines for intravitreal injection. For these and other reasons, eye drops are particular preferred.

Thus, in one embodiment, the polypeptide is comprised in a composition suitable for use as eye drops.

Said eye drops are foreseen for administration to the mammalian subject. Eye drops are foreseen for use on the ocular route to administer the agent of the present invention. Preferably, the eye drops are administered topically to the eye.

Methods for making eye drops are known in the art. Without limitation and for illustration only, methods for making eye drops have been described in WO 2016/162812 A1.

In some embodiments, the polypeptide is comprised in a composition, such as an eye drop composition, comprising additionally one or more carriers and/or one or more excipients. The term "carrier" as used herein refers to an organic or inorganic component, of a natural or synthetic nature, which is combined together with the active ingredient in order to enable, enhance or facilitate application of the active ingredient. The term "excipient" as used herein is intended to indicate all substances which may be present in a pharmaceutical composition of the present invention and which are not active ingredients such.

Preferably the composition according to the present invention comprises at least water as an excipient. In some embodiments, the composition according to the present invention comprises aqueous media, and more preferably the composition according to the present invention is in the form of an aqueous solution. In one embodiment, the polypeptide is comprised in an aqueous medium, and the aqueous medium is administered to the mammalian subject. The aqueous medium may be for example an aqueous solution. Aqueous solutions and other respective compositions, in some embodiments, are obtainable directly from purification of NGF in aqueous media. For example, when the agent according to the present invention is obtained by purification form a biological source by purification, respective aqueous compositions may be obtainable directly from the last purification step, e.g. elution from the last chromatographic column (usually the polishing step) and/or filtration. Alternatively, respective compositions are available through an additional step of adjustment to final protein concentration and/ preparation of a desired formulation. Such additional step may include, for example, a step of clarification or filtration, as described herein, and/or addition of one or more excipients and/or one or more carriers. Exemplary compositions useful in the present invention are described herein, without limitation.

Thus, the polypeptide may be present in a composition, e.g. in a pharmaceutical composition. The compositions described herein are preferably sterile and preferably contain the polypeptide as a pharmaceutically active peptide or protein, and optionally of further agents, mentioned or not mentioned herein. The compositions may be in any state, e.g. liquid, frozen, lyophilized, etc.

The compositions described herein may comprise salts, buffer substances, preservatives, carriers, diluents and/or excipients, all of which are preferably pharmaceutically acceptable. The term "pharmaceutically acceptable" describes something non-toxic and/or which does not interact with the action of the active ingredient of the pharmaceutical composition.

Suitable buffer substances for use in the invention include acetic acid in a salt, citric acid in a salt, boric acid in a salt and phosphoric acid in a salt. For example, it is preferable that the polypeptide of the invention, as a result of the various aspects of the present invention, obtainable in a buffer having a pH between 4.5 and 6.5, preferably between 5.0 and 6.0. In one embodiment, an acetate buffer is a suitable buffer for such purposes, and is therefore particularly preferred. Thus, in one embodiment, the polypeptide of the invention is obtained in an acetate buffer having a pH between 4.5 and 6.5, preferably between 5.0 and 6.0. In particular, it is considered that acetate is an optimal buffer to stabilize NGF and derivatives thereof in the pH range of pH 5.0 to pH 5.8.

Since NGF, and likely also the polypeptides according to the present invention, is sensitive to oxidation at the methionine residues, methionine is preferably comprised in the formulation.

The present disclosure also concerns a polypeptide of SEQ ID NO: 4, wherein the composition is characterized by a pH of pH 5.0 to 6.0 (preferably pH 5.5), and comprises the following:
a) 0.2 to 20 mg/ml of said polypeptide (preferably 2 mg/ml),
b) 5 to 100 mM sodium acetate buffer (preferably 20 mM),
c) 5 to 100 mM methionine (preferably 20 mM).

Thus, preferably, the polypeptide is comprised in a composition comprising the following:
a) 0.2 to 20 mg/ml of the polypeptide of the present invention,
b) 5 to 100 mM sodium acetate buffer,
c) 5 to 100 mM methionine,
d) pH 5.0 to 6.0.

A more preferred pH range is 5.0 to 5.8.

Most preferably, the polypeptide is comprised in a composition comprising the following:
a) 2 mg/ml of said polypeptide,
b) 20 mM mM sodium acetate buffer,
c) 20 mM methionine,
d) pH 5.5.

Another preferred formulation within the present invention is the following:
1 mg/ml polypeptide of SEQ ID NO: 4 (preferred)
10 mM Na acetate buffer
10 mM Methionine
154 mMNaCl
0,1 mg/mL Polysorbate 80
pH 5.5
These and other formulations are envisaged to be suitable as drug product for ocular use.

The above compositions are preferably eye drops. The above compositions are preferably aqueous compositions.

The composition (formulation) according to the present invention may be conserved, without limitation, according to one or more of the following embodiments:
- First embodiment: the above formulation can be stored frozen at -70 degrees C (data not shown), and thawed before administration.
- Second embodiment: the above formulation can be stored in the refrigerator, preferably in the temperature range of +2 to + 8 degrees C (data not shown).
- Third embodiment: the above formulation can be subjected to drying or freeze-drying, and then stored, e.g. at room temperature (data not shown). It can be reconstituted before administration.

All the above embodiments provide certain advantages of the freezing at e.g. -20 degrees C, as this avoids the use of dry ice shipping, the necessity to thaw and remix post-thawing, etc. In such embodiments, the formulation according to the present invention overcomes certain inconveniences of state of the art pharmaceutical NGF formulations, such as oxervate (cenegermin) in particular.

A particularly preferred composition for administration according to the present invention is exemplified in Example 3.

Suitable preservatives for use in the compositions according to the present invention include those known in the art, among which are for illustration but without limitation benzyl alcohol, benzalkonium and its salts, M-cresol, phenol, chlorobutanol, paraben and thimerosal. These and other preservatives are optionally included in the composition according to the present invention.

Thus, the present invention provides polypeptide of SEQ ID NO: 3 (which is not part of the present invention) or SEQ ID NO: 4 for therapeutic use, i.e. for the use in a method of treatment of the human or animal body by therapy. Therapy may include prevention and/or treatment of a condition. In view of the potential for therapeutic use, said polypeptide can also be referred to as a pharmaceutically active protein or peptide.

Optionally the administration according to the present invention is accompanied by administration of at least one antimicrobial agent, such as an antibiotic. The antimicrobial agent may be part of the composition comprising the polypeptide according to the present invention, or alternatively may be administered to the subject separately, to the same or a different site, by the same or a different route of administration.

### INDUSTRIAL APPLICABILITY

The polypeptide of SEQ ID NO: 3 (not part of the invention) or SEQ ID NO: 4 described herein is suitable for a variety of purposes, e.g. for therapeutic applications as described herein.

The following examples and figures are intended to illustrate some preferred embodiments of the invention and should not be interpreted to limit the scope of the invention, which is defined by the claims.

### EXAMPLES

### MATERIALS AND METHODS COMMON TO MORE THAN ONE EXAMPLE

Unless specified otherwise, the following experimental examples concern specifically the polypeptide of SEQ ID NO: 4 characterized, with respect to wild-type human NGF, by the substitutions P61S R100E (termed "NGF P61S R100E", Malerba et al. PLOS One, 2015, vol. 10, e0136425, SEQ ID NO: 4), as well as pro-forms etc. thereof. The polypeptide of SEQ ID NO: 4 may also be referred to as "NGF mutein", but it has to be borne in mind that the therapeutic suitability, specific for this protein, according to this invention, and as demonstrated in the experimental examples, particularly Example 4, is remarkably different from wild-type human NGF. Likewise, as described in Example 2, purification of the polypeptide of SEQ ID NO 4 differs from published purification protocols for wild-type NGF, and the specific process for preparing said polypeptide according to the present invention is suitable for achieving high purity, in particular absence of des-nona variant and trypsin.

The polypeptide of SEQ ID NO: 4 was recombinantly expressed as a precursor. To that end, SEQ ID NO: 4 was fused to the pro-peptide of wild-type human NGF (positions 1-121 of SEQ ID NO: 1). In other words, the precursor of the polypeptide of SEQ ID NO: 4 consisted of the precursor of human wild-type NGF (SEQ ID NO: 1), except for the substitutions P61S R100E in the mature portion of human wild-type NGF (but, for clarity, lacking the 2 most C-terminal amino acids of SEQ ID NO: 1 which do not form part of the polypeptide sequence of human wild-type NGF). Expression was performed in *E. coli* Rosetta (DE3) (strain: *E.coli* Rosetta (DE3) / pET11a-hpro NGF P61S R100E), in the form of insoluble inclusion bodies.

### Equipment

**Table 1 List of Equipment used.**

| **Device** | **Inventory** | **Serial No.** | **Supplier** |
|---|---|---|---|
| 1 L Bioreactors (incl. sensors and pumps) | E023, E024 | 07462/09, 07463/09 | Sartorius Stedim |
| 10 L Bioreactor | E082 | - | Sartorius Stedim |
| 300 V Power Source | E018; E019 | - | VWR |
| Äkta Explorer100a | E011 | 001054 | GE Healthcare |
| Äkta Explorer100a | E054 | 18111241 | GE Healthcare |
| Autoclave Systec VX-120 | E050 | 2512 | Systec GmbH |
| Centrifuge Galaxy 14D | E016 | 904090 | VWR |
| Centrifuge Sorvall Evolution RC | F683 | - | Sorvall |
| Clean bench | E006 | 40970929 | Thermo |
| Electrophoresis chamber Novex Mini Cell | - | - | Invitrogen |
| High pressure homogenizer APV 2000 | F688 | 5-07.791 | APV |
| HPLC, 1100 Series | E053 | - | Agilent |
| Magnetic stirrer MR Hei-Mix S | E013 | 30948231 | Heidolph |
| Magnetic stirrer PC-620D | 686 | - | Corning |
| pH-Meter inlab pH720 | E017 | 9080718 | WTW |
| Photometer Genesys 10uv | E051 | 2L9Q013008 | Thermo |
| Pipetus | - | - | Hirschmann Laborgeräte |
| Pump VL 1000 | F606 | 0208004 | Verder |
| Scale | F651 | - | Sartorius |
| Scale | E030 | W092934 | Kern |
| Scale | E009 | - | Mettler |
| Shaker IKA KS 4000ic | E049 | - | IKA |
| Vortexer | E012 | 40934086 | VWR |

### Protein parameters of proteins and peptides described herein

Theoretical values for protein parameters of relevant proteins were calculated with ExPASy's ProtParam-Tool, which is available at http://web.expasy.org/protparam. These are shown in Table 2, as follows:

**Table 2: Theoretically deduced properties of relevant proteins/polypeptides**

| | | | |
|---|---|---|---|
| | pro-form of SEQ ID NO: 4 | SEQ ID NO: 4 | porcine Trypsin |
| **MW** monomeric | 24.8 kDa | 13.23 kDa | 24.4 kDa |
| pI | 9.7 | 8.2 | 7.0 |
| ε | 25168 l / mol / cm | 19668 l / mol / cm | 34295 l / mol / cm |

### Analytical Methods

### SDS-PAGE and Western Blot

SDS-PAGE and Western Blots were performed using standard procedures. For SDS-PAGE, 12 % Bis-TRIS NuPAGE gels (Article No. NP0342BOX from Thermo Fisher) were operated under reducing conditions at constant Volt (175 V) in NuPAGE MES-running buffer (Article No. NP0002 from Thermo Fisher). The primary antibody for Western Blot was purchased from Santa Cruz Biotechnology (NGF (H-20) sc-548). Examples of results are shown e.g. in Fig. 9A and Fig. 10.

### Analytical CEX-HPLC

CEX-HPLC was performed using a ProPac SCX-10 from Dionex. The column was operated with 50 mM citrate buffer, pH 5.5 at 1 mL/min. For elution, 1 M NaCl (B) was added and a linear gradient over 50 minutes from 0-100 % B was executed. An example of results is shown in Fig. 9B.

### SE-HPLC

SE-HPLC was performed using a Superdex 200 Increase 10/300 GL from GE Healthcare. The column was operated in PBS. Product was detected at 280 nm.

### Endotoxin, DNA and HCP

Endotoxin, DNA and host cell proteins (HCP) were determined according to standard protocols.

### EXAMPLE 1: EXPRESSION OF THE POLYPEPTIDE OF SEQ ID NO: 4 AS A PRECURSOR PROTEIN

### Production Strain

The gene encoding for pro-NGF was cloned to pET11a expression plasmid. The gene was derived from *H. sapiens* and two point mutations (namely P61S and R100E) were introduced into the open reading frame. Subsequently, chemical competent Rosetta *(DE3)* cells were transformed with the expression plasmid and a single colony was selected (the resulting strain was termed E5901-STRAIN (= E.coli Rosetta (DE3) / pET11a-pro NGF P61S R100E

NGF RCB C-151101)). Aliquots were stored at <-60°C, in 1.0 mL.

In Example 1, initial fermentation development based on the strain E5901-STRAIN is described.

### Equipment

| **Device** | **Inventory-** | **Serial No.** | **Supplier** |
|---|---|---|---|
| Autoclave Systec VX-120 | E050 | 2512 | Systec GmbH |
| Centrifuge Galaxy 14D | E016 | 904090 | VWR |
| Centrifuge Sorvall Evolution RC | F683 | - | Sorvall |
| Clean bench | E006 | 40970929 | Thermo Scientific |
| Magnetic stirrer MR Hei-Mix S | E013 | 30948231 | Heidolph |
| Magnetic stirrer PC-620D | 686 | - | Corning |
| pH-Meter inlab pH720 | E017 | 9080718 | WTW |
| Photometer Genesys 10uv | E051 | 2L9Q013008 | Thermo Spectronics |
| Pipetus | - | - | Hirschmann Laborgeräte |
| Shaker IKA KS 4000ic | E049 | - | IKA |
| Weight Kern 572 | E030 | W092934 | Kern |
| Weight Mettler AE160 | E009 | - | Mettler |
| 1 L Bioreactors (incl. sensors and pumps) | E023, E024 | 07462/09, 07463/09 | Sartorius Stedim |

### Growth Media

### Complex medium for fermentation

The complex medium used for fermentation was composed of: 49.3 g/L yeast extract, 0.61 g/L MgSO₄*7H₂O, 0.5 g/L NH₄Cl, 14.2 g/L K₂HPO₄*3H₂O and 10 g/L glucose. The feed used for this fermentation was composed of 263 g/L yeast extract and 133 g/L glucose.

### Minimal Media (MM) for fermentation

| **Constituent** | **MM I - Final conc. [mM]** | **MM II - Final conc. [mM]** |
|---|---|---|
| Aluminum chloride, hexahydrate | N/A | 0.000063 |
| Ammonium sulfate | 39.4 | N/A |
| Boric acid | 0.005 | 0.000125 |
| Calcium chloride, dihydrate | 2 | 0.000875 |
| Citric acid, monohydrate | 25.2 | 10 |
| Cobalt(II) chloride, hexahydrate | N/A | 0.00075 |
| Cobalt(II) sulfate, heptahydrate | 0.014 | N/A |
| Copper(II) sulfate, pentahydrate | 0.032 | 0.00425 |
| Diammonium phosphate | N/A | 35 |
| Dipotassium phosphate | N/A | 45 |
| Disodium hydrogen phosphate | 7.5 | N/A |
| Ferric chloride, hexahydrate | 0.37 | 0.17 |
| Kanamycin | 0.103 | 0.103 |
| Magnesium sulfate, heptahydrate | 4 | 3 |
| Manganese(II) sulfate, monohydrate | 0.142 | 0.00375 |
| Polypropylene glycol 2000 | N/A | N/A |
| Potassium chloride | 53.6 | N/A |
| Sodium chloride | 8.5 | 40 |
| Sodium dihydrogen phosphate, monohydrate | 31.9 | N/A |
| Sodium molybdate, dihydrate | 0.001 | 0.00005 |
| Zinc sulfate, heptahydrate | 0.073 | 0.000375 |

For the batch phase, both basic media were supplemented with 30 g/L glucose. If not stated otherwise, the feed had the same composition as the respective batch medium, but contained 300 g/L of the respective carbon-source.

### LB-agar plates with Ampicillin and Chloramphenicol

LB-agar plates were freshly poured. The medium was composed of 10 g/L peptone, 5 g/L yeast extract, 5 g/L NaCl and 15 g/L agar. After autoclaving, the medium was supplemented with 100 µg/ml ampicillin and 30 µg/mL chloramphenicol.

### Fermentation

If not stated otherwise, fermentation, in this Example 1, fermentation was performed in 1 L stirred glass bioreactors controlled by a Biostat B unit from Sartorius. Typically, pO₂ was controlled to 30 %, cultivation temperature was set to 37 °C and pH was controlled to 7 using 2 M phosphoric acid and 25 % ammonium hydroxide. Unless stated otherwise, the batch phase was followed by an exponential feed with F₀= 6 g/L/h and µ= 0.25 /h. For practical reasons, all exponential feeds were approximated by two linear feeds. Typically, induction of product expression was executed by addition of 1 mM IPTG and after induction, a constant feed rate of 10 g/L/h was applied. Cell biomass was harvested by centrifugation using a Sorvall Evolution RC from Thermo Scientific. The centrifuge was equipped with a SLC-6000 rotor and the culture was centrifuged at 8500 rpm and 4 °C for 30 min.

### Relative quantification of product in biomass samples

At given time-points, culture samples were diluted to an OD₆₀₀ of 10 and the biomass from 100 µL aliquots of this dilution was pelleted. Pellets were resuspended in 150 µl (non-reducing) Laemmli buffer and samples were boiled for 5 min. at 95°C. 10 µL of each sample were analyzed on a 10 % Bis-Tris gel from Novex. Electrophoretic separation was performed for 90 min at 125 V and gels were stained with Coomassie. Destained gels were scanned and the abundance of the band corresponding to the precursor of the polypeptide of SEQ ID NO: 4 was quantified by densitometry. To further correct for variabilities in utilized biomass, the intensity of the band corresponding to the precursor of the polypeptide of SEQ ID NO: 4 was normalized to the intensity of a housekeeping protein.

Relative product accumulation was calculated from the increase of the band corresponding to the precursor of the polypeptide of SEQ ID NO: 4, post and pre-induction. Notably, the measured value represents a specific yield (i.e. normalized to an OD₆₀₀=10). For the absolute yield of a given fermentation, the actual cell-density has to be included in the consideration (cf. below).

### Absolute quantification of product in biomass samples

A standard for the precursor of the polypeptide of SEQ ID NO: 4 was obtained from the European Brain Research Institute (EBRI, Rome, Italy). The standard was diluted to a concentration of 65 µg/mL in Laemmli-buffer. The stated protein concentration was defined by EBRI. A standard curve was prepared with 260, 520, 780, 1040 and 1300 ng of the standard for the precursor of the polypeptide of SEQ ID NO: 4. Samples were analyzed on the same gel as the standard curve and dilution factor of the sample was considered to calculate the absolute product yield of product at the given time.

### Summary and Conclusions of Example 1

Based on the above, it is concluded that the production strain (E5901-STRAIN, cf. above) was successfully used for fermentation in 1 L scale. While different media compositions have been assessed for their ability to promote bacterial growth and product expression, minimal medium MM I supplemented with 5 g/L yeast extract proved to be favorable in terms of expression yield and obtainable cell density. In terms of product formation, no significant differences were observed, when the main culture was performed either with or without antibiotics (Ampicillin and Chloramphenicol, data not shown).

Example 1 can be up-scaled in order to produce the polypeptide at industrial scale.

### EXAMPLE 2: LAB-SCALE PURIFICATION, ESTABLISHMENT OF CAPTO MMC

The precursor of SEQ ID NO: 4, used in this Example, was obtained in inclusion bodies as described in Example 1.

### Starting point for optimization in view of the state of the art

At the onset, it was reasoned that a process for improved purification of the polypeptide of the present invention could, in the absence of indications to the contrary, follow the basic cornerstones of NGF purification as previously reported in the literature. However, it was also borne in mind that, for an efficient production at large scale, adaptations suitable for a later scale up should be considered. Thus, it has been reasoned, based on Rattenholl et al. (supra), on WO2013092776 A1, and on other publications, that the polypeptide of SEQ ID NO: 4 may likewise be obtained at least at a lab-scale process, via its pro-form, employing unspecific digestion using trypsin and subsequent purification. It was reasoned that highly pure mature polypeptide of SEQ ID NO: 4 could be obtained thereby. However, it is only through the specific adaptions and modifications reported in this example that highly pure mature polypeptide of SEQ ID NO: 4 was obtained. Therefore, the administration of a polypeptide of SEQ ID NO: 4 to a subject in need thereof is enabled particularly in view of the high purity of the polypeptide of SEQ ID NO: 4 as described herein.

### Equipment production of the polypeptide of SEQ ID NO: 4

| **Device** | **Inventory-No.** | **Serial No.** | **Supplier** |
|---|---|---|---|
| 1 L Bioreactors (incl. sensors and pumps) | E023, E024 | 07462/09, 07463/09 | Sartorius Stedim |
| 300 V Power Source | E018; E019 | - | VWR |
| Äkta Explorer100a | E011 | 001054 | GE Healthcare |
| Äkta Explorer100a | E054 | 18111241 | GE Healthcare |
| Autoclave Systec VX-120 | E050 | 2512 | Systec GmbH |
| Centrifuge Galaxy 14D | E016 | 904090 | VWR |
| Centrifuge Sorvall Evolution RC | F683 | - | Sorvall |
| Clean bench | E006 | 40970929 | Thermo Scientific |
| Electrophoresis chamber Novex Mini Cell | - | - | Invitrogen |
| High pressure homogenizer APV 2000 | F688 | 5-07.791 | APV |
| HPLC, 1100 Series | E053 | - | Agilent |
| Magnetic stirrer MR Hei-Mix S | E013 | 30948231 | Heidolph |
| Magnetic stirrer PC-620D | 686 | - | Corning |
| pH-Meter inlab pH720 | E017 | 9080718 | WTW |
| Photometer Genesys 10uv | E051 | 2L9Q013008 | Thermo Spectronics |
| Pipetus | - | - | Hirschmann Laborgeräte |
| Pump VL 1000 | F606 | 0208004 | Verder |
| Scale | F651 | - | Sartorius |
| Scale | E030 | W092934 | Kern |
| Scale | E009 | - | Mettler |
| Shaker IKA KS 4000ic | E049 | - | IKA |
| Vortexer | E012 | 40934086 | VWR |

| | | | |
|---|---|---|---|
| List of Equipment used in Example 2. | | | |

Details of the manufacturing process according to this Example, including improvements described in Example 2B, are given in the process overview in Fig. 1.

Unless specified otherwise, analytical methods were as described in the above section "Analytical methods".

### Example 2A: purification based on previously described protocols.

*E. coli* cells expressing the precursor of the polypeptide of SEQ ID NO: 4 ("biomass") were produced as described in Example 1, and cells were lysed by addition of lysozyme and subsequent sonication on ice. Inclusion bodies ("IBs") were (1) extracted from the host cells and washed with 6% Triton X100 (in 1.5 M NaCl, 60 mM EDTA) and , and (2) solubilized in 6 M guanidinium HCl ("gHCl"), 0.1 M Tris-HCl pH 8.0, 1 mM EDTA, 100 mM (fresh) DTT. IBs were solubilized for 2h at room temperature. Afterwards, the pH was lowered to 3-4 by addition of 37 % HCl. The thus obtained solution comprising solubilized precursor of the precursor of the polypeptide of SEQ ID NO: 4 ("solubilizate") was dialyzed against 6 M gHCl (pH 3-4).

Refolding of the precursor of the polypeptide of SEQ ID NO: 4 was performed in 0.1 M Tris-HCl, 1 M L-arginine, 5 mM EDTA, 0.61 g/L oxidized glutathione and 1.53 g/L reduced glutathione, pH 9.5 at +4°C. Therefore, 50 µg of protein were added per mL of refold buffer, each hour. After refolding, the reaction was dialyzed against 50 mM sodium phosphate pH 7.0. While the buffer was exchanged, significant precipitation occurred.

The precursor of the polypeptide of SEQ ID NO: 4 was purified over a consecutive sequence of cation-exchange chromatography (SP Sepharose HP operated with 50 mM sodium phosphate, pH 7.0 and eluted with a NaCl-gradient) and subsequent hydrophobic-interaction chromatography (Phenyl Sepharose HP, operated with 50 mM sodium phosphate, 1 M ammonium sulfate pH 7.0). Afterwards, another dialysis was employed to exchange the sample's buffer against 50 mM sodium phosphate, pH 7.0 (note that such second dialysis could, however, be omitted in the process). Again significant amounts of product did precipitate throughout the process of reduction of the buffer's conductivity.

The thus prepared precursor of the polypeptide of SEQ ID NO: 4 was subjected to limited proteolysis by adding 1 mg trypsin per 250 mg pro-NGF. The exposure of the precursor of the polypeptide of SEQ ID NO: 4 to the protease was for 14h at 2-8°C.

The matured NGF was finally polished over a cation-exchanger (SP Sepharose XL operated with 50 mM sodium phosphate, pH 7.0 and eluted with a NaCl-gradient). Finally, product was concentrated to 0.5 - 1 mg/mL and was frozen at <-65°C.

### Example 2B: Improvements

In the following, several improvements, compared to Example 2A, as tested and implemented in the course of arriving at the present invention, are described. Unless the context dictates otherwise, all those details which are not expressly indicated were as described above for Example 2A.

### Optimization of IB-solubilization

While low amounts of IBs (as exemplary received from shaking flask cultures) had been previously reported to be readily solved in the solubilization buffer (6 M gHCl, 0.1 M Tris-HCL pH 8.0, 1 mM EDTA, 100 mM (fresh) DTT), the IBs obtained from high cell-density fermentations could not be resolved entirely. This could be solved herein by addition of 2 M urea to said solubilization buffer, which turned out to improve the solubilization yield significantly (data not shown). For the avoidance of doubt: the 2 M urea were present in addition to the 6 M gHCl and other ingredients.

### Refolding optimization

It was decided, initially based on Rattenholl et al. (2001, Eur. J. Biochem, vol. 268, p. 3296-3303; Rattenholl, 2001, Dissertation zur Erlangung des akademischen Grades doctor rerum naturalium (Dr. rer. nat.), Martin-Luther-Universität Halle-Wittenberg (Germany)), but importantly also taking into consideration a later (up)scalability, to perform the refolding with 200 to 500 mg of the precursor of the polypeptide of SEQ ID NO: 4 per liter of refolding reaction, preferably 200 to 300 mg of the precursor of the polypeptide of SEQ ID NO: 4 per liter of refolding reaction. This lead to relatively good yield of solubilized precursor of the polypeptide of SEQ ID NO: 4. In particular it important to consider that by such increased amount of NGF compared to the volume of refolding reaction, and under consideration that the refolding reaction comprises relatively expensive ingredients such as glutathione and arginine, relatively more the precursor of the polypeptide of SEQ ID NO: 4 could be refolded per volume of refolding reaction, which should render the refolding economically feasible, also at production scale.

### Purification of the precursor of the polypeptide of SEQ ID NO: 4

Purification of the precursor of the polypeptide of SEQ ID NO: 4, after refolding, was done by an approach which utilizes the rather high isoelectric point of pro-NGF and employs a cation exchange stationary phase (namely SP Sepharose) for purification. In order to run this type of chromatography, for technical reasons, the refolding buffer has to be exchanged against a buffer with low conductivity. While doing this, significant quantities the precursor of the polypeptide of SEQ ID NO: 4 precipitated (data not shown). This observation could be attributed to the reduction of the arginine concentration in the buffer.

Therefore, some efforts were taken to replace the capture column by a different one (a column with different selectivity), which could be more tolerant to the presence of arginine in the refolding reaction. In a first attempt to do this, the performance of several r stationary phases was assessed, but none of the approaches resulted in promising results (cf. Table 6). Therefore, the stationary phase used for the capture column was kept as it was defined by the previous process. However, due to the high isoelectric point (pI) of the precursor of the polypeptide of SEQ ID NO: 4, an increase in the conductivity of the running buffer (by addition of 250 mM L-arginine) was possible without affecting the performance. By this, refolded precursor of the polypeptide of SEQ ID NO: 4 could be stabilized to a certain extent and the amount of precipitated precursor was reduced (data not shown).

**Table above: Alternative selectivities tested for capture of the precursor of the polypeptide of SEQ ID NO: 4, and evaluation thereof.**

| **Potential capture step** | **Short description** | **Evaluation** |
|---|---|---|
| Hydrophobic interaction chromatography | Hydrophobic interaction chromatography is not susceptible to conductivity and salt composition of the loaded sample. | If ammonium sulfate or sodium chloride were added to product present in the refolding buffer, protein precipitated heavily. Even addition of small quantities of ammonium sulfate (to a concentration of 0.25 M) led to precipitation. Also addition of sodium chloride was not possible without provoking precipitation of product. Therefore, a capture step based on Phenyl- or Butyl-Sepharose is not an option. |
| Mixed-mode chromatography | Capto MMC is a mixed-mode stationary phase combining hydrophobic properties with those of a stationary phase for cation-exchange. Because binding is not solely mediated by ionic interactions, this stationary phase is more salt-tolerant than classical cation-exchange stationary phase. | Within an initial set-up, a phosphate buffer at pH 5.5 was supplemented with 0.25 M L-Arginine and elution was facilitated with a NaCl-gradient. However, no significant amounts of product (precursor) were recovered by this approach. |
| Size exclusion chromatography | Size exclusion chromatography has a good resolution and is independent from the sample-buffer. The typical bottleneck of this type of chromatography (i.e. its limited capacity) is not applicable since relatively low amounts of product are requested. | Although the preparative chromatogram looked promising, no separation of the precursor of the polypeptide of SEQ ID NO: 4 from impurities could be achieved. |
| | | This may be caused by the existence of this specific precursor as a polydisperse mixture under the investigated conditions. |

Regarding mixed mode chromatography, it is understood, however without wishing to be bound to a particular theory, that the precursor of the polypeptide of SEQ ID NO: 4 does not elute efficiently from mixed mode chromatography, whereas the mature polypeptide of SEQ ID NO: 4 does.

### Protease digestion to yield mature NGF

For manufacture of the polypeptide of SEQ ID NO: 4, the protease (trypsin) is essential and therefore, it was reasoned that ideally, the particular trypsin selected should meet the following criteria:
1. Derived from a recombinant source. Certification of animal-free raw material is pivotal for later on required GMP-compliance of the process.
2. Low side-activity of trypsin. Notably, trypsin can be subjected to autolysis. This process may result in so called pseudotrypsin, which has a broadened substrate-spectrum and possesses chymotrypsin-like activity. Ca²⁺ (e.g. 1 mM CaCl2) may be added to reduce autolysis. However, nowadays typically "modified trypsin" is applied for every protocol, which requires a tight sequence specificity (e.g. for peptide finger printing). This modified trypsin is typically obtained by acylation of trypsin's exposed ε-amino groups of lysine residues.
3. Low batch-to-batch variability, in order to enable a reproducible production process. Alternatively, the chosen enzyme should be delivered with a certificate stating the specific activity of the respective batch. The required amount of enzyme may then be based on activity rather than on mass.

Despite a comprehensive search, no trypsin fulfilling both criteria 1 and 2 was identified on the commercial market. It was reasoned that criterion 1 is more important. To reduce autolysis, addition of CaCl₂ may be sufficient. As a result, a recombinant 'GMP grade' trypsin from Roche (Roche 06369880103, Lot: 11534700) was chosen as raw material for the process. The sequence of this enzyme, which is expressed in *Pichia pastoris,* was derived from *Sus scrofa.* According to its certificate, the utilized trypsin batch has a specific activity of 4997 U/mg (determined according to USP).

### Omission of a second purification step prior to trypsinization

Within an initial screen searching for optimal enzyme/substrate ratios for the intended trypsinization, the precursor of the polypeptide of SEQ ID NO: 4 obtained from the capture column (see above) was used. In contrast to a previously established process (European Brain Research Institute (EBRI), details not published, based on Rattenholl et al., *supra*) it was decided not to use an additional hydrophobic interaction chromatography prior to trypsinization. The decision to omit such a second column purification step prior to trypsinization was mainly based on two lines of thinking: On the one hand, product obtained after the capture column was already virtually pure according to SDS-PAGE. On the other hand, the trypsinization itself may help to improve the impurity-profile by digestion of remaining host cell proteins (HCPs).

Table 7 summarizes the matrix of conditions screened within the first round. Results of trypsinization were investigated 12 % SDS-PAGE (data not shown). The results (data not shown) indicate that trypsinization reproducibly yields stable polypeptide of SEQ ID NO: 4 over a rather wide range of enzyme/substrate-ratios (i.e. from 1 - 5 µg trypsin per 375 µg precursor of the polypeptide of SEQ ID NO: 4). Timing of digestion is not highly critical. Therefore, stopping of the reaction and the time required to load the reaction to the polishing column is apparently not limiting. This finding is of special importance since the reaction cannot be suitably or economically quenched at preparative scale.

Some additional experiments were conducted in order to refine an optimal enzyme/substrate ratio for the envisaged trypsinization, and it was found that with an enzyme/substrate ratio of 1/100 to 1/200 (protein weight/protein weight), good yields of the polypeptide of SEQ ID NO: 4 on the one hand and low amounts of truncated products on the other hand could be obtained reproducibly. It has to be stated that under the utilized conditions (i.e. within phosphate/arginine buffer (pH 7.0) at 2-8°C and incubated (exposed to protease) for two to six hours) the quality of the digestion was not highly dependent on the enzyme/substrate ratio. This finding is of special importance since the underlying enzymatic digestion is prone to minor variations in the experimental set-up (e.g. alteration of trypsin's activity due to batch-to-batch variability or storage of the enzyme; timing and temperature of the incubation step (exposure to protease); errors in determination of protein concentrations). Moreover, this is also the reason why extended fine-tuning at small scale to further reduce potential truncation products seems to be not meaningful. If an "optimal" condition would be identified in small scale, there is still a good chance to produce a slightly changed product-pattern the next time virtually the same digest is repeated at larger scale.

### Polishing chromatography with the aim to obtain pure polypeptide, following trypsinization

In contrast to a previously established process (European Brain Research Institute (EBRI), details not published, based on Rattenholl et al., *supra*), which employed a SP Sepharose stationary phase for polishing of mature polypeptide (note: SP sepharose is a cation exchange stationary phase), here a more suitable stationary phase was searched for, based on the following considerations: In order to be efficiently loaded to an SP Sepharose column, a reduction of the conductivity of the solution comprising the precursor of the polypeptide of SEQ ID NO: 4, such as by buffer exchange is required. It is however known (e.g. Example 2A) that reduction of the ionic strength of the solution does result in precipitation of the target molecule and therefore, a buffer exchange to a low conductivity buffer should be avoided. Moreover, a cation exchange stationary phase was already used for capture of the precursor of the polypeptide of SEQ ID NO: 4, and an orthogonal selectivity is preferred in order to achieve a better separation of remaining contaminants. A third and final argument against the use of a SP stationary phase for purification of the trypsinization reaction is that potentially remaining precursor of the polypeptide of the precursor of the polypeptide of SEQ ID NO: 4 would bind to this column and could be separated from mature polypeptide of SEQ ID NO: 4 merely by elution selectivity and not by binding selectivity.

In order to establish such an orthogonal polishing column for purification of mature polypeptide of SEQ ID NO: 4, it was intended to use a hydrophobic interaction (HIC) column in a first instance. This stationary phase was not only chosen to have an orthogonal selectivity, but also because a buffer exchange to a low conductivity buffer is not necessary. Despite testing of several HIC stationary phase and conditions (e.g. Phenyl- and Butyl-Sepharose operated with 1 M (NH₄)₂SO₄ and 0.5 M (NH₄)₂SO₄, respectively), no satisfying polishing step based on HIC could be implemented (data not shown).

However, in a further experimental setup for a polishing step, the mixed mode stationary phase Capto MMC was tested and could be implemented successfully. It was found that with optimized conditions, the stationary phase binds to the polypeptide of SEQ ID NO: 4 reversibly and the product can be eluted by increasing pH (data not shown). In contrast, the precursor of the polypeptide of SEQ ID NO: 4 binds irreversibly onto the stationary phase and can be only eluted by using 1M NaOH as mobile phase (data not shown). Furthermore, it could be shown that trypsin does not bind at all onto the column operated at the same conditions (data not shown). These results provide clear evidence that the Capto MMC stationary phase is capable of efficiently separating mature polypeptide of SEQ ID NO: 4 from trypsin and from remaining precursor of the polypeptide of SEQ ID NO: 4.

### Establishment of an additional membrane chromatography

In order to further deplete endotoxins and DNA, an additional anion-exchange membrane was included in the process. In general, and as is commonly known, membrane chromatography is characterized in that a solution comprising a component to be analyzed or purified (in the present case the polypeptide of SEQ ID NO: 4) is passed over or through a membrane, which is normally charged. For that purpose, in the present case a STIC-membrane (Sartorius, Goettingen, Germany) was incorporated at the positions indicated in Fig. 1. It could be shown that the polypeptide of SEQ ID NO: 4 does not bind to the membrane, and thus, a proof of concept was provided that membrane chromatography is suitable for purification of the polypeptide of SEQ ID NO: 4. For illustration of the incorporation in the entire process, including membrane chromatography, see Fig. 1.

### Reproducibility of the process according to Example 2

In order to probe the robustness of the process, the process was conducted five times and resulting fractions were analyzed with respect to their yield and purity. Throughout these runs, a steady optimization of process details was pursued and buffer composition, gradients and so on were adopted until the final, optimized process details (see Fig. 1) were established. The results indicate that in lab-scale approx. 50 to 100 mg polypeptide of SEQ ID NO: 4 can be yielded from one consistent production run. Notably, the product obtained was consistently found, by SDS polyacrylamide gel electrophoresis followed by Coomassie staining or silver staining, to be relatively pure (less than five percent of contaminating host cell proteins and only traces of truncated NGF, data not shown).

For the precursor of the polypeptide of SEQ ID NO: 4 no meaningful method for SE-HPLC could be established. In contrast, SE-HPLC analysis for mature polypeptide of SEQ ID NO: 4 was straightforward and resulted in a homogeneous product peak of approx. 16 kDa which fits with a monomeric state of the polypeptide of SEQ ID NO: 4 n (data not shown).

### Summary and Conclusions

For this process, refolded precursor of the polypeptide of SEQ ID NO: 4 was captured using a SP Sepharose FF ("FF" stands for Fast Flow, i.e. a stationary phase with relatively large particles) and was subsequently treated with trypsin to yield mature NGF. For that purpose, the arginine concentration of the refolding reaction was decreased from 1 M (as recommended by the prior art) to 350 mM.

Control of the proteolytic cleavage of the precursor of the polypeptide of SEQ ID NO: 4 to yield mature polypeptide of SEQ ID NO: 4 is considered as most critical factor for the process. Herein, conditions were identified to reproducibly facilitate cleavage with high efficiency on the one hand and prevent formation of degradation products of NGF. The experimental data herein have shown that an apparently robust production process can be established over a rather wide range of enzyme/substrate ratios. For the trypsinization, step yields are apparently good and no significant loss is expected at this stage of the process. The product pattern obtained does apparently not strongly depend from the used reaction conditions (in terms of enzyme/substrate-ratio and time of incubation (time of exposure to protease)). Notably, even if a good yield for polishing of the enzyme is expected, at least 2*x grams of the polypeptide of SEQ ID NO: 4 have to be processed in order to deliver x gram of mature polypeptide of SEQ ID NO: 4.

The purification according to this example is a lean process consisting of merely two chromatographic purification steps. The existing purification process was further optimized and several aspects were adopted for scale-up (see Fig. 1). Exemplary, previously used methods of cell disruption were replaced by high-pressure homogenization and all dialysis steps could be replaced by tangential-flow filtration. The thus established process is capable to deliver the polypeptide of SEQ ID NO: 4 at high purity.

Despite the named challenges, the overall process is capable of delivering product of acceptable quality. In particular, the process yields the polypeptide of SEQ ID NO: 4 in a state that is essentially free of degradants of the polypeptide, in particular essentially free of the des-nona variant of the polypeptide. Thus, the process yields the polypeptide at high purity.

The complete process incorporating the improvements according to Example 2, including membrane chromatography, is schematically depicted in Fig. 1.

Example 2 can be up-scaled in order to produce the polypeptide at industrial scale.

### Example 3: Preparation of a formulation according to the present invention

A formulation comprising the polypeptide of SEQ ID NO: 4, previously prepared according to Example 2, was prepared in consideration of the suggestions of Eng et al., 1997, Anal. Chem., vol. 69, p. 4184-4190.

In particular, the formulation prepared has the following composition:
2 mg/mL polypeptide according to SEQ ID NO: 4, obtained as described in Example 2
20 mM Na acetate buffer
20 mM Methionine
pH 5.5

In particular, it is considered that acetate is a suitable buffer to stabilize NGF and derivatives thereof in the pH range of pH 5.0 to pH 5.8. Since NGF, and likely also the polypeptides according to the present invention, is sensitive to oxidation at the methionine residues, methionine is comprised in the formulation.

The following embodiments work, inter alia, for conservation of the formulation:
- First embodiment: the above formulation can be stored frozen at -70 degrees C (data not shown), and thawed before administration.
- Second embodiment: the above formulation can be stored in the refrigerator, preferably in the temperature range of +2 to + 8 degrees C (data not shown).
- Third embodiment: the above formulation can be subjected to drying or freeze-drying, and then stored, e.g. at room temperature (data not shown). It can be reconstituted before administration.

All the above embodiments provide certain advantages of the freezing at e.g. -20 degrees C, as this avoids the use of dry ice shipping, the necessity to thaw and remix post-thawing, etc. In such embodiments, the formulation according to the present invention overcomes certain inconveniences of state of the art pharmaceutical NGF formulations, such as oxervate (cenegermin) in particular.

### Example 4: Proof of concept in non-human mammals

The aim of this Example is to investigate the efficacy of the polypeptide of SEQ ID NO: 4 for the treatment and/or prevention of ophthalmic disorders in non-human animals.

The present invention is, in part, based on experiments with animal models.

Reported herein is a study of administration of the polypeptide of SEQ ID NO: 4 to non-human animals. The polypeptide of SEQ ID NO: 4 is obtainable at high purity by expression as described in Example 1 and purification as described in Example 2. It was formulated as described in Example 3.

### Material and Methods

### Optic nerve crush (ONC) model

Rats (Sprague Dawley, male, 180-200 g, Charles River, Italy) were housed in a temperature- and humidity-controlled vivarium (12 h dark/light cycle, free access to food and water). Behavioral experiments were performed in a quiet, temperature-controlled room (20 to 22°C) between 9 a.m. and 5 p.m. by an operator blinded to the status of drug treatment.

To perform optic nerve crush (ONC), rats were anaesthetized with a mixture of ketamine and xylazine (90 mg/kg and 3 mg/kg, respectively, intraperitoneal, i.p.) and the optic nerve was accessed by an incision in the conjunctiva temporally around the eye. The left optic nerve was crushed at 1 mm from the optic disk for 10 seconds, applying a constant and consistent force using cross-action forceps. All procedures were performed on the left eye under aseptic conditions. ONC was induced in the left experimental eye, while the right eye served as an internal control. Before and after the procedure, the eye fundus was observed through the operating microscope to assess the integrity of the retinal blood flow.

In a first experimental setting, retinas were dissected at day 4, 7 and 14 after the ONC and subjected to retinal ganglion cells immunodetection to assess a time course of the retina damage. Untouched and untreated contralateral right eyes were used as control for ONC-induced loss retinal ganglion cell. Day 7 after ONC was selected as optimal time-point for the initial investigation of the effect of the polypeptide of SEQ ID NO: 4, since approximately 50% of the retinal ganglion cell population was present.

Rats were subjected to drug treatment after intravitreal injection and eye drops application following a rescue treatment schedule in order to investigate the effect of the polypeptide of SEQ ID NO: 4 in an experimental setting potentially translatable to the clinic:
- treatment with vehicle or the polypeptide of SEQ ID NO: 4: 20, 2, 0.2 µg/ml solution; volume of 2 µl intravitreal injection starting at day 4 after injury and lasting until day 7.
- treatment with vehicle or the polypeptide of SEQ ID NO: 4: 200 µg/ml solution; volume of 25 µl eye drop starting at day 4 after injury and lasting until day 7, once, twice or three times a day.

After treatment, retinas were dissected and subjected to retinal ganglion cells immunodetection to quantify retinal damage and the effect of the different treatments with the polypeptide of SEQ ID NO: 4.

### Immunohistochemistry

Dissected retinas were post-fixed in formalin for 24 hours and paraffin embedded. Some formalin fixed paraffin-embedded sections (5 µm) were stained with hematoxylin and eosin (H&E) for histological examination. Other sections were subjected to deparaffinization and rehydration by sequential incubations in xylene and descending ethanol series (100%, 90% and 70%). Antigen retrieval was achieved by microwaving the sections in 10 mM citrate buffer (pH 6.0) for 5 minutes. Sections were incubated with the primary antibodies (Brn3a, 1:500; and RBPMS 1:500) 1 hour in a humidity chamber. Sections were then incubated with prediluted biotinylated anti-mouse/rabbit IgG secondary antibody or with biotinylated anti-guinea pig secondary antibody (1:200, #BA-7000, Vector Laboratories, Burlingame, US), 30 minutes at room temperature, and in avidin-biotin complex solution (#PK-7200, Vectastain Elite ABC-HRP Kit, Vector Laboratories, Burlingame, US) for 30 minutes at room temperature. The sections were then transferred to peroxidase substrate (#K3468, ImmPACT DAB, Vector Laboratories, Burlingame, US) for 4-6 minutes for the chromogen development reaction and rinsed in distilled water before mounting. Nuclei were counterstained with Mayer's hematoxylin. Tissues were visualized, and digital images were captured using an optical microscope Leica DM2500 (Leica Microsystems, Milan, Italy).

After selection at low power magnification of immunopositive cells, quantification of total cell, Brn3a+ and RBPMS+ cells were done in 7.104 µm2 boxes at high-power fields (HPF, X400, Leica Microsystem). For each rat, 3 different sections for each eye were analyzed.

Data (means ± SEM) were compared using a one-way ANOVA followed by post hoc comparisons using the Bonferroni correction. GraphPad Prism (version 5.00, La Jolla, USA) was used for all analysis and P < 0.05 was considered statistically significant.

### Results

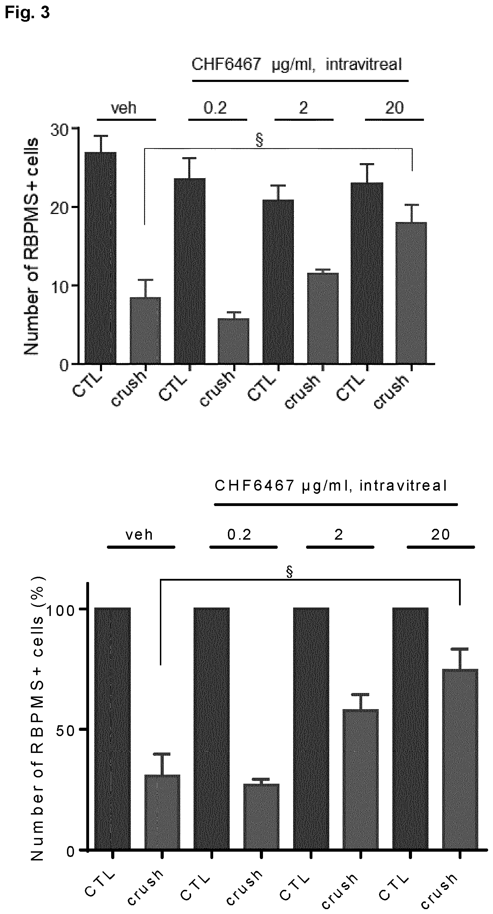

In rats submitted to intravitreal treatment with vehicle starting at day 4 after ONC, in agreement with data generated, during the set-up of the model, a decrease of about 60% in the number of retinal ganglion cells was observed. The polypeptide of SEQ ID NO: 4 intravitreally administered at 3 different dose levels (20, 2, 0.2 µg/ml solution; injection volume of 2 µl) exerted a dose-dependent protective effect on retinal ganglion cells loss that reached statistical significance in rats receiving intravitreal injections with the 20 µg/ml solution. In this group, the number of retinal ganglion cells in the eye subjected to the ONC was similar to the healthy contralateral eye (Fig. 3).

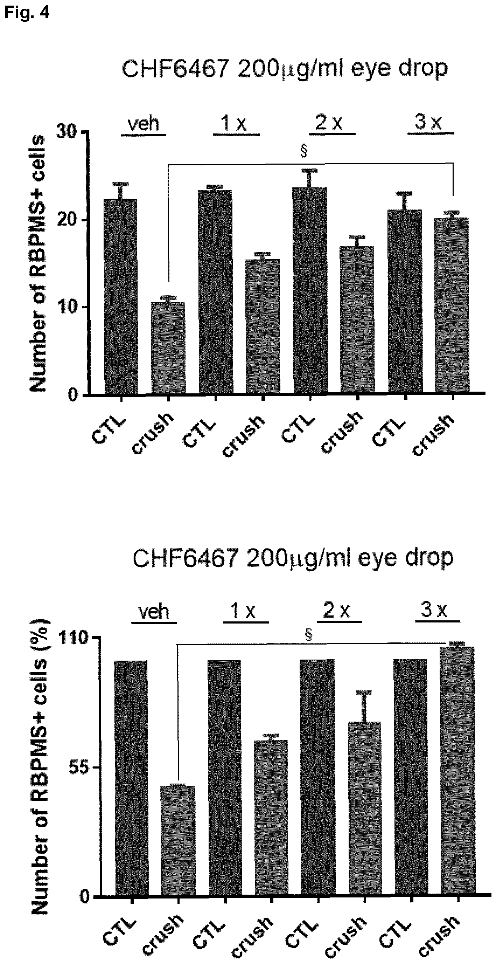

Also, in the groups of rats submitted to ONC and vehicle eye drops treatment starting at day 4 after injury, the loss in retinal ganglion cells was super-imposable to the one quantified in the set-up phase of the model and in the experiment evaluating the effect of the polypeptide of SEQ ID NO: 4 after intravitreal injection. The rescue treatment effect of eye drops of the polypeptide of SEQ ID NO: 4 (200 µg/ml) was investigated by applying 3 different treatment schedules: once, twice or three times a day. The data generated show a significantly higher biological activity of the 200 µg/ml solution given three times a day, even if a trend for efficacy is also evident after administration once and twice daily (Fig. 4).

Conclusion: The rescue effects of the polypeptide of SEQ ID NO: 4, when given four days after optic nerve damage is completely unexpected and open the possibility that the compound could be effective in functional recovery of patients with optic nerve and other retinal disorders for traumatic, ischemic, inflammatory, metabolic or neoplastic reasons, whenever RGC loss is part of the pathological process.

### Example 5: Comparison between the polypeptide of SEQ ID NO: 2 and the polypeptide of SEQ ID NO: 4 in eliciting nociceptive behaviour and facial allodynia after ocular administration in mice

The aim of this Example is to investigate the efficacy of the polypeptide of SEQ ID NO: 4 for the treatment and/or prevention of ophthalmic disorders in non-human animals.

The present invention is, in part, based on experiments with animal models.

Reported herein is a study of administration of the polypeptide of SEQ ID NO: 4 to non-human animals. The polypeptide of SEQ ID NO: 4 is obtainable at high purity by expression as described in Example 1 and purification as described in Example 2. It was formulated as described in Example 3.

### Aims of this Example:

1. The present example aims to compare the algogenic activity of the polypeptide of SEQ ID NO: 4 to wild-type human NGF (polypeptide of SEQ ID NO: 2) and wild-type mouse NGF (mNGF) after ocular instillation in a mouse model of constriction of infraorbital nerve (CION).
2. The effect of the NGF analog (polypeptide of SEQ ID NO: 4) will be evaluated by measuring their ability to induce acute nociceptive responses after ocular instillation in comparison with polypeptide of SEQ ID NO: 2, mNGF and the irritant reference compound capsaicin, know to induce a nociceptive response. The following doses are being tested: 0.5, 1, 5 and 10 µg in 5 µl/eye, diluted in isotonic saline (NaCl 0.9%). Capsaicin will be tested at 0.001-0.5 nmol/5µl/eye.
3. A sub-threshold dose of the different compounds will be then tested in their ability to induce a nociceptive response after the ocular instillation in the Constriction of the Infraorbital Nerve (CION) model.

### Materials and Methods

### Murine NGF (mouse NGF, mNGF)

murine NGF was high purity native mouse NGF 2.5S (>95%) and was obtained by extraction and purification from mouse submaxillary glands according to the method described by Bocchini et al., 1969, Pro.c Natl. Acad. Sci. USA, vol. 64, p. 787-794. mNGF consists of residues 129-241 of the polypeptide sequence of UniProt P01139.

### In vivo models of nociception

Animal experiments will be carried out according to the European Union (EU) guidelines for animal care procedures and the Italian legislation (DLgs 26/2014) application of the EU Directive 2010/63/EU. Studies will be conducted under University of Florence research permits #194/2015-PR. C57BL/6 mice (male, 25-30 g, Envigo, Milan, Italy) will be used for nociceptive tests. Animals will be housed in a temperature- and humidity-controlled vivarium (12 h dark/light cycle, free access to food and water). Behavioral experiments will be performed in a quiet, temperature-controlled room (20 to 22 °C) between 9 a.m. and 5 p.m. by an operator blinded to the status of drug treatment.

### Constriction of the Infraorbital Nerve (CION)

CION will be performed in C57BL/6 mice as reported (Vos et al., 1994, J. Neurosci., vol. 14, p. 2708-2723; Luiz et al., 2010, Neuropeptides, vol. 44, p. 87-92) Briefly, mice will be anesthetized with an intraperitoneal (i.p.) injection of a mixture of ketamine (90 mg/kg) and xylazine (3 mg/kg) and an incision will be made in the left upper lip skin lateral to the nose, and the rostral end of the infraorbital nerve was exposed. Then, two loosely constrictive ligatures (#6/0 silk suture) will be placed around the infraorbital nerve with a distance of 2 mm. In the sham procedure, the left infraorbital nerve will be exposed but not ligated. Neomycin sulfate and sulfathiazole (powder, 0.05 g and 9.95 g, respectively; Boehringer Ingelheim Italia S.p.A, Italy) will be applied to the wound and the incision will be sutured. Mice will be monitored, adequately rehydrated, and maintained in a controlled temperature (37°C) until fully recovered from anesthesia. All the experiment will be performed at day 10 after surgery. At the end of the experiments, animals will be euthanized with inhaled CO2 plus 10-50% O2.

### Eye wiping assay in mice

Ocular instillation (5 µl) of test compounds, polypeptide of SEQ ID NO: 4, polypeptide of SEQ ID NO: 2, mNGF, (all, 0.5, 1, 5 and 10 µg in 5 µl/eye) and capsaicin (0.5 nmol/5µl/eye) or their respective vehicles (isotonic saline, NaCl 0.9% and 1% dimethyl sulfoxide, DMSO) will be used to induce an acute nociceptive response as previously described (De Petrocellis et al., 2010, Pharmacol. Res., vol. 63, p. 294-299). Mice will be placed individually inside a Plexiglas chamber and will be acclimatized for 20 minutes before stimulus. The number of eye wiping movements following the drug instillation into the eye will be recorded for a 5-minutes time-period and will be considered as index of pungency.

At day 10 after CION or sham procedure, mice will receive an ocular instillation (5µl/eye) of a sub-threshold dose of polypeptide of SEQ ID NO: 2, mNGF and polypeptide of SEQ ID NO: 4, or capsaicin and the nociceptive response will be measured.

### Results

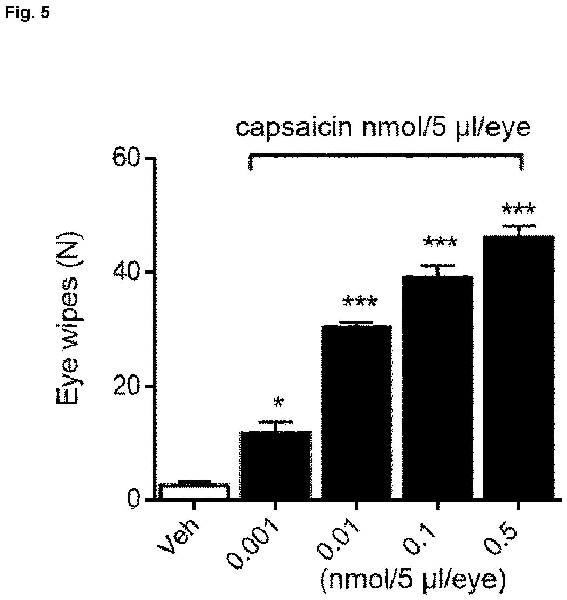

In the first part of the example, tested different doses were tested (0.001-0.5 nmol) of capsaicin administered by ocular instillation (5 µl/drop eye), in their ability to induce the acute nociceptive response, measured as number of eye wipes in 5-minutes time-period. Capsaicin induced a dose dependent nociceptive response as demonstrated by the increase in the eye wipes responses measured after the ocular instillation of capsaicin (0.001-0.5 nmol/5 µl/eye) (Fig. 5).

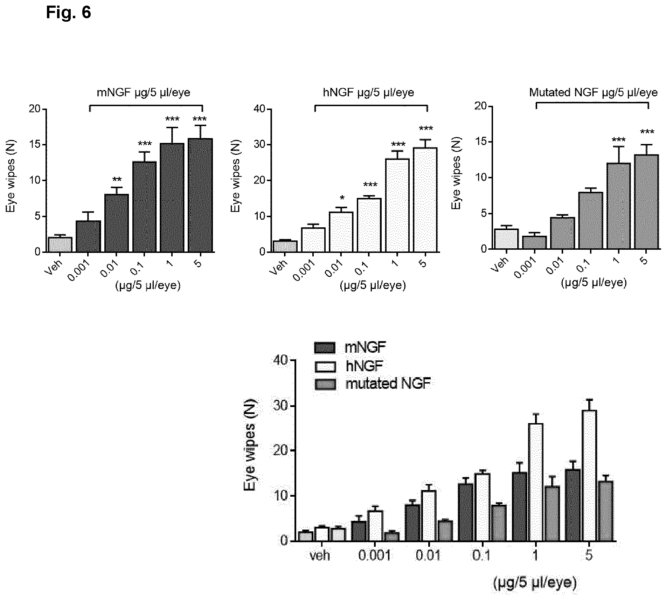

Next, different doses were evaluated (0.001-5 µg 5 µl/eye) of mouse NGF (mNGF), human NGF (polypeptide of SEQ ID NO: 2) and the polypeptide of SEQ ID NO: 4). All compounds induced a dose-dependent increase in the nociceptive response measured as the number of eye wipes following ocular application. The application of the polypeptide of SEQ ID NO: 2 showed more potency to induce a nociceptive response compared to the mNGF and more importantly when compared to the mutated form of the polypeptide of SEQ ID NO: 2 (Fig. 6).

Next, the effect of a sub-threshold dose of the different compounds in a model of Constriction of the Infraorbital Nerve (CION) was evaluated. The CION model induced a sensitization to further nociceptive stimuli (Trevisan et al., 2016, Brain, 139 (Pt 5), p.1361-1377).

At day 10 after CION or sham procedure, mice received an ocular instillation of sub-threshold dose of capsaicin (0.001 nmol/5µl/eye), mNGF (0.001 nmol/5µl/eye), polypeptide of SEQ ID NO: 2 (0.001 nmol/5µl/eye), and polypeptide of SEQ ID NO: 4 (0.001 nmol/5µl/eye) the eye wiping nociceptive response will be measured.

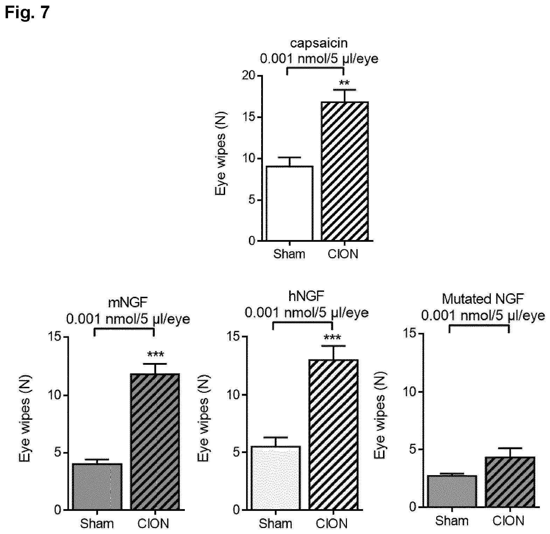

Data showed that in a sensitized model (CION model) the nociceptive responses produced by sub-threshold dose of capsaicin evoked a more intense response in CION operated mice than in sham-operated mice (Fig. 7). Same results were obtained when the mNGF and polypeptide of SEQ ID NO: 2 were instilled in the eye of CION-operated mice. The ocular instillation of the polypeptide of SEQ ID NO: 4 failed to induce an increase in the nociceptive response.

### Example 6: Use for treating or preventing ophthalmologic disorders in humans

The aim of this Example is to further support the efficacy of the polypeptide of SEQ ID NO: 4 for the treatment and/or prevention of ophthalmic disorders in humans.

Suitable dosages of the polypeptide can be determined by the person skilled in the art based on the guidance given herein.

The inventors expect to use in humans the same concentrations found to be effective in the rats with optic nerve injury (for eye drops 200 µg/mL three time per day) for topical administration, in particular. Most preferred is conjunctival administration.

### BRIEF DESCRIPTION OF THE FIGURES

**Fig. 1****:** Outline of the process according to Example 2, including improvements described in Example 2B.
**Fig. 2****:** Polypeptide sequences. Asterisk (*) = position 61 in mature human NGF; cross (+): position 100 in mature human NGF.
   **A:** SEQ ID NO: 1: Sequence of pre-pro human NGF as encoded by the respective human Open Reading Frame.
      Pre-peptide: amino acid positions 1-18; pro-peptide: amino acid positions 19-121; mature NGF: amino acid positions 122-239; C-terminal dipeptide: amino acid positions 240-241.
      Disulfide bonds (in the correctly folded mature part): linking amino acid positions 136 ↔ 201,
         179 ↔ 229, 189 ↔ 231.
      Furin cleavage site (RSKR): amino acid positions 118-121.
   **B:** Schematic overview of pre-peptide, pro-peptide and mature NGF.
   **C:** SEQ ID NO: 2: Sequence of mature human NGF.
   **D:** SEQ ID NO: 3 (not part of the present invention)
   **E:** SEQ ID NO: 4
**FIG. 3****:** EFFECT OF THE POLYPEPTIDE OF SEQ ID NO: 4 ("CHF 6467") AFTER INTRAVITREAL ADMINISTRATION. STATISTICAL ANALYSIS BY ONE-WAY-ANOVA FOLLOWED BY BONFERRONI POST-HOC ANALYSIS. N = 6/GROUP.
**FIG. 4****:** EFFECT OF THE POLYPEPTIDE OF SEQ ID NO: 4 AFTER TREATMENT WITH EYE DROPS. STATISTICAL ANALYSIS BY ONE-WAY-ANOVA FOLLOWED BY BONFERRONI POST-HOC ANALYSIS. N = 3/GROUP (FOR DETAILS SEE EXAMPLE 4).
**Fig. 5****:** Dose-dependent eye wiping response evoked by ocular instillation (5 µl/drop eye) of capsaicin (0.001-0.5 nmol) in C57BL/6 mice. Error bars indicate mean ± SEM, 6-8 mice per group. Veh is the vehicle of CPS. *P< 0.05, ***P< 0.001 vs. Veh. Oneway ANOVA with Bonferroni posthoc correction.
**Fig. 6****:** Dose-dependent eye wiping response evoked by ocular instillation (5 µl/drop eye) of mouse NGF (mNGF), polypeptide of SEQ ID NO: 2 ("human NGF") and the polypeptide of SEQ ID NO: 4 ("mutated NGF") (0.001-0.5 nmol) in C57BL/6 mice. Error bars indicate mean ± SEM, 6-8 mice per group. Veh is the vehicle of mNGF, polypeptide of SEQ ID NO: 2 and polypeptide of SEQ ID NO: 4. *P< 0.05, ***P< 0.001 vs. Veh. Oneway ANOVA with Bonferroni post-hoc correction.

## Claims

1. A polypeptide of SEQ ID NO: 4 for use in treatment and/or prevention of an ophthalmic disorder in a mammalian subject, wherein the ophthalmic disorder involves a damage and/or disorder of the optic nerve or the ophthalmic disorder involves a damage and/or disorder of retinal ganglion cells.

2. The polypeptide for use according to claim 1, wherein the mammalian subject is a human.

3. The polypeptide for use according to any one of the preceding claims for administration to the eye.

4. The polypeptide according to claim 3, wherein the administration is selected from the group consisting of topical administration to the eye and intravitreal administration, whereby topical administration is preferred.

5. The polypeptide for use according to any one of the preceding claims, wherein the polypeptide is administered repeatedly.

6. The polypeptide for use according to claim 5, wherein the polypeptide is administered repeatedly at least three times per day.

7. The polypeptide for use according to claim 5 or claim 6, wherein the polypeptide is administered repeatedly, for a period of three to 30 days, preferably seven to 14 days.

8. The polypeptide for use according to any one of the preceding claims, wherein the polypeptide is administered following a damage of the optic nerve.

9. The polypeptide for use according to claim 8, wherein the polypeptide is administered at least four days following the damage of the optic nerve.

10. The polypeptide for use according to any one of the preceding claims, wherein the damage and/or disorder of the optic nerve comprises at least one selected from the group consisting of glaucoma, neurotrophic keratitis, optic neuritis, optic nerve atrophy, optic nerve head drusen and optic pathway glioma.

11. The polypeptide for use according to any one of the preceding claims, wherein the dose/each dose has an amount of 0.3 to 30 µg of the polypeptide per eye, more preferably 1 to 10 µg of the polypeptide per eye, and most preferably 5 µg of the polypeptide per eye.

12. The polypeptide for use according to any one of the preceding claims, wherein the polypeptide is comprised in an aqueous medium, and the aqueous medium is administered to the mammalian subject.

13. The polypeptide for use according to claim 12, wherein said polypeptide is comprised in a composition comprising the following:
a) 0.2 to 20 mg/ml of said polypeptide (preferably 2 mg/ml),
b) 5 to 100 mM sodium acetate buffer (preferably 20 mM),
c) 5 to 100 mM methionine (preferably 20 mM),
d) pH 5.0 to 6.0 (preferably pH 5.5).

14. The polypeptide for use according to any one of the preceding claims, wherein the polypeptide is essentially free of degradants of the polypeptide, in particular essentially free of the des-nona variant of the polypeptide.

15. The polypeptide for use according to any one of the preceding claims, wherein the polypeptide is obtainable by recombinant expression and purification, wherein the purification comprises purification on a mixed mode stationary phase.

## Patentansprüche

1. Polypeptid von SEQ ID NO: 4 zur Verwendung bei der Behandlung und/oder Prävention einer Augenerkrankung bei einem Säugetier als Probanden, wobei die Augenerkrankung eine Schädigung und/oder Störung des Sehnervs oder die Augenerkrankung eine Schädigung und/oder Störung der retinalen Ganglienzellen beinhaltet.

2. Polypeptid zur Verwendung nach Anspruch 1, wobei das Säugetier ein Mensch ist.

3. Polypeptid zur Verwendung nach einem der vorhergehenden Ansprüche zur Verabreichung am Auge.

4. Polypeptid nach Anspruch 3, wobei die Art der Verabreichung aus der Gruppe, bestehend aus topischer Verabreichung am Auge und intravitrealer Verabreichung, ausgewählt wird, wobei eine topische Verabreichung bevorzugt wird.

5. Polypeptid zur Verwendung nach einem der vorhergehenden Ansprüche, wobei das Polypeptid wiederholt verabreicht wird.

6. Polypeptid zur Verwendung nach Anspruch 5, wobei das Polypeptid wiederholt mindestens dreimal pro Tag verabreicht wird.

7. Polypeptid zur Verwendung nach Anspruch 5 oder Anspruch 6, wobei das Polypeptid wiederholt für einen Zeitraum von drei bis 30 Tagen, vorzugsweise für sieben bis 14 Tage, verabreicht wird.

8. Polypeptid zur Verwendung nach einem der vorhergehenden Ansprüche, wobei das Polypeptid nach einer Schädigung des Sehnervs verabreicht wird.

9. Polypeptid zur Verwendung nach Anspruch 8, wobei das Polypeptid für mindestens vier Tage nach der Schädigung des Sehnervs verabreicht wird.

10. Polypeptid zur Verwendung nach einem der vorhergehenden Ansprüche, wobei die Schädigung und/oder Störung des Sehnervs mindestens eines umfasst, das in der folgenden Gruppe aufgeführt ist: Glaukom, Neurotrophe Keratopathie, Optikusneuritis, Optikusatrophie, Drusenpapille und Optikusgliom.

11. Polypeptid zur Verwendung nach einem der vorhergehenden Ansprüche, wobei die Dosis/jede Dosis eine Menge von 0,3 bis 30 µg des Polypeptids pro Auge, mehr bevorzugt 1 bis 10 µg des Polypeptids pro Auge und am meisten bevorzugt 5 µg des Polypeptids pro Auge aufweist.

12. Polypeptid zur Verwendung nach einem der vorhergehenden Ansprüche, wobei das Polypeptid in einem wässrigen Medium enthalten ist und das wässrige Medium dem Säugetier verabreicht wird.

13. Polypeptid zur Verwendung nach Anspruch 12, wobei das Polypeptid in einer Zusammensetzung enthalten ist, die Folgendes umfasst:
a) 0,2 bis 20 mg/ml des Polypeptids (vorzugsweise 2 mg/ml),
b) 5 bis 100 mM Natriumacetat-Puffer (vorzugsweise 20 mM),
c) 5 bis 100 mM Methionin (vorzugsweise 20 mM),
d) pH 5,0 bis 6,0 (vorzugsweise pH 5,5).

14. Polypeptid zur Verwendung nach einem der vorhergehenden Ansprüche, wobei das Polypeptid im Wesentlichen frei ist von Abbaustoffen des Polypeptids, insbesondere im Wesentlichen frei von der Des-Nona-Variante des Polypeptids ist.

15. Polypeptid zur Verwendung nach einem der vorhergehenden Ansprüche, wobei das Polypeptid durch rekombinante Expression und Reinigung erhalten werden kann, wobei die Reinigung die Reinigung an einer stationären Phase mit gemischtem Modus umfasst.

## Revendications

1. Polypeptide présentant la SEQ ID NO: 4 pour une utilisation dans le traitement et/ou la prévention d'un trouble ophtalmique chez un sujet mammifère, dans laquelle le trouble ophtalmique implique une lésion et/ou un trouble du nerf optique, ou le trouble ophtalmique implique une lésion et/ou un trouble des cellules ganglionnaires de la rétine.

2. Le polypeptide destiné à être utilisé selon la revendication 1, dans laquelle le sujet mammifère est un humain.

3. Le polypeptide destiné à être utilisé selon l'une quelconque des revendications précédentes pour une administration dans l'oeil.

4. Polypeptide selon la revendication 3, dans laquelle l'administration est choisie dans le groupe constitué par l'administration topique dans l'oeil et l'administration intravitréenne, moyennant quoi l'administration topique est préférée.

5. Polypeptide destiné à être utilisé selon l'une quelconque des revendications précédentes, dans laquelle le polypeptide est administré de manière répétée.

6. Polypeptide destiné à être utilisé selon la revendication 5, dans laquelle le polypeptide est administré de manière répétée au moins trois fois par jour.

7. Polypeptide destiné à être utilisé selon la revendication 5 ou la revendication 6, dans laquelle le polypeptide est administré de manière répétée, pendant une période de trois à 30 jours, de préférence de sept à 14 jours.

8. Polypeptide destiné à être utilisé selon l'une des revendications précédentes, dans laquelle le polypeptide est administré à la suite d'une lésion du nerf optique.

9. Polypeptide destiné à être utilisé selon la revendication 8, dans laquelle le polypeptide est administré au moins quatre jours après la lésion du nerf optique.

10. Polypeptide destiné à être utilisé selon l'une des revendications précédentes, dans laquelle la lésion et/ou le trouble du nerf optique comprend au moins un élément choisi dans le groupe constitué par le glaucome, la kératite neurotrophique, la névrite optique, l'atrophie du nerf optique, le drusen de la tête du nerf optique et le gliome des voies optiques.

11. Polypeptide destiné à être utilisé selon l'une quelconque des revendications précédentes, dans laquelle la dose/chaque dose a une quantité de 0,3 à 30 µg du polypeptide par oeil, de préférence de 1 à 10 µg du polypeptide par oeil, et de préférence 5 µg du polypeptide par oeil.

12. Polypeptide destiné à être utilisé selon l'une quelconque des revendications précédentes, dans laquelle le polypeptide est compris dans un milieu aqueux, et le milieu aqueux est administré au sujet mammifère.

13. Polypeptide destiné à être utilisé selon la revendication 12, dans laquelle ledit polypeptide est compris dans une composition comprenant ce qui suit :
a) 0,2 à 20 mg/ml dudit polypeptide (de préférence 2 mg/ml) ;
b) 5 à 100 mM de solution tampon d'acétate de sodium (de préférence 20 mM) ;
c) 5 à 100 mM de méthionine (de préférence 20 mM) ;
d) pH de 5,0 à 6,0 (de préférence pH de 5,5).

14. Polypeptide destiné à être utilisé selon l'une quelconque des revendications précédentes, dans laquelle le polypeptide est essentiellement exempt de produits de dégradation du polypeptide, en particulier essentiellement exempt de la variante des-nona du polypeptide.

15. Polypeptide destiné à être utilisé selon l'une quelconque des revendications précédentes, dans laquelle le polypeptide peut être obtenu par expression et purification de protéines recombinantes, dans laquelle la purification comprend la purification sur une phase stationnaire en mode mixte.
